# EUROPEAN PATENT SPECIFICATION

(11) **EP 4 294 939 B1**
(45) Date of publication and mention of the grant of the patent: **12.11.2025**
(21) Application number: 22757063.7
(22) Date of filing: 21.02.2022
(51) Int. Cl.: C12Q 1/68, C07K 14/81

(54) **METHODS FOR IDENTIFICATION AND TREATMENT OF BLEEDING IN ANIMALS**
VERFAHREN ZUR IDENTIFIZIERUNG UND BEHANDLUNG VON BLUTUNGEN BEI TIEREN
PROCÉDÉS POUR L'IDENTIFICATION ET LE TRAITEMENT DES HÉMORRAGIES CHEZ LES ANIMAUX

(30) Priority: 22.02.2021 US 202163152219 P
(43) Date of publication of application: 27.12.2023
(73) Proprietor: Washington State University, Pullman, WA 99164-1060 (US); Scottish Deerhound Club of America, Deerfield, WI 53531-9717 (US)
(72) Inventor: COURT, Michael H., Pullman, Washington 99164-1060 (US); DILLBERGER, John E., Nashville, IN 47448 (US)
(74) Representative: HGF
(86) International application number: PCT/US2022/017130
(87) International publication number: WO 2022/178343

(56) References cited:
- EP-A1- 3 412 776
- EP-B1- 3 124 619
- WO-A1-2013/156785
- WO-A1-2016/118723
- US-A1- 2008 057 491
- US-A1- 2010 062 981
- US-A1- 2010 144 880
- US-A1- 2015 148 406
- BENT LIND ET AL: "A novel missense mutation in the human plasmin inhibitor (alpha2-antiplasmin) gene associated with a bleeding tendency", BRITISH JOURNAL OF HAEMATOLOGY, JOHN WILEY, HOBOKEN, USA, vol. 107, no. 2, 24 December 2001 (2001-12-24), pages 317 - 322, XP071120455, ISSN: 0007-1048, DOI: 10.1046/J.1365-2141.1999.01708.X
- JAIN SHILPA ET AL: "Inherited disorders of the fibrinolytic pathway", TRANSFUSION AND APHERESIS SCIENCE, ELSEVIER SCIENCE, LONDON, GB, vol. 58, no. 5, 8 August 2019 (2019-08-08), pages 572 - 577, XP085944327, ISSN: 1473-0502, [retrieved on 20190808], DOI: 10.1016/J.TRANSCI.2019.08.007
- LAW RUBY H P ET AL: "HEMOSTASIS, THROMBOSIS, AND VASCULAR BIOLOGY X-ray crystal structure of the fibrinolysis inhibitor _ 2 -antiplasmin", 15 February 2017 (2017-02-15), pages 2049 - 2052, XP055773847, Retrieved from the Internet <URL:https://watermark.silverchair.com/zh800408002049.pdf?token=AQECAHi208BE49Ooan9kkhW_Ercy7Dm3ZL_9Cf3qfKAc485ysgAAA8kwggPFBgkqhkiG9w0BBwagggO2MIIDsgIBADCCA6sGCSqGSIb3DQEHATAeBglghkgBZQMEAS4wEQQMFlUIpyzSR2d0VANOAgEQgIIDfNdXrImboz2VE3hNMtF04ZnLCfCXoBtfqMaPwHYDKwW7boGx0_i6STkUEYQ2fpfYDB0owI06yfOH8YCS5reAr> [retrieved on 20210209], DOI: 10.1182/blood-2007-
- CHRISTIANSEN VICTORIA J. ET AL: "Hemostasis, Thrombosis, and Vascular Biology: The effect of a single nucleotide polymorphism on human [alpha]2-antiplasmin activity - PMC", vol. 109, no. 12, 15 June 2007 (2007-06-15), US, pages 5286 - 5292, XP093199836, ISSN: 0006-4971, Retrieved from the Internet <URL:https://www.ncbi.nlm.nih.gov/pmc/articles/PMC1890835/> DOI: 10.1182/blood-2007-01-065185
- ROY SWAPAN: "The Functional Subproteomes of Serpin Protease Inhibitors are Now Open for LC-MS Biomarker Discovery", MOJ PROTEOMICS & BIOINFORMATICS, vol. 3, no. 6, 5 August 2016 (2016-08-05), XP055964882, DOI: 10.15406/mojpb.2016.03.00106
- WIINBERG B., JENSEN A.L., JOHANSSON P.I., ROZANSKI E., TRANHOLM M., KRISTENSEN A.T.: "Thromboelastographic Evaluation of Hemostatic Function in Dogs with Disseminated Intravascular Coagulation", JOURNAL OF VETERINARY INTERNAL MEDICINE, WILEY-BLACKWELL PUBLISHING, INC., US, vol. 22, no. 2, 1 March 2008 (2008-03-01), US , pages 357 - 365, XP055964890, ISSN: 0891-6640, DOI: 10.1111/j.1939-1676.2008.0058.x
- LADAS ET AL.: "Topical Yunnan Baiyao administration as an adjunctive therapy for bleeding complications in adolescents with advanced cance r", SUPPORT CARE CANCER, vol. 20, 2012, pages 3379 - 83, XP037121045, DOI: 10.1007/s00520-012-1598-1

## Description

### REFERENCE TO SEQUENCE LISTING SUBMITTED ELECTRONICALLY

The official copy of the sequence listing is submitted electronically via EFS-Web as an ASCII formatted sequence listing with a file named 350778_ST25.txt created on February 18, 2022 and having a size of 40.8 kilobytes and is filed concurrently with the specification. The sequence listing comprised in this ASCII formatted document is part of the specification.

### BACKGROUND AND SUMMARY OF THE INVENTION

Bleeding in animals is recognized as a significant health problem, especially in canines. In particular, several dog breeds can experience delayed bleeding in post-operative settings, including sighthound dog breeds such as racing greyhounds and Scottish deerhounds. This syndrome was first identified in retired racing greyhounds in 2007, indicating that bleeding disorders were one of the four most commonly reported causes of death in this breed, with a significant proportion of those deaths attributed to postoperative bleeding.

A subsequent prospective clinical study of greyhounds undergoing routine gonadectomy (ovariohysterectomy or castration) demonstrated unexpected postoperative bleeding in 26% of dogs, with unexplained bleeding observed 36 to 48 hours after the procedure. Signs of abnormal bleeding ranged from severe skin bruising around the surgical site to frank oozing of blood from the wound that was accompanied by a greater than 6% decrease in hematocrit. There was no evidence for bleeding at sites distant from the wound.

Although bleeding can spontaneously resolve without the need for transfusion of blood products a comparison of a range of measures of primary and secondary hemostasis in blood samples from affected and unaffected dogs failed to show any abnormality. However, whole blood thromboelastography suggests excessive fibrinolysis (hyperfibrinolysis), including poor clot strength, and lower plasma antiplasmin activity in affected dogs.

It has been suggested that the antifibrinolytic drug epsilon aminocaproic acid (EACA) could be utilized to prevent delayed postoperative bleeding. Over a 5-year period in 46 greyhounds that underwent limb amputation for osteosarcoma, 67% of greyhounds (4/6) experienced bleeding prior to introduction of prophylactic treatment. After introduction of prophylactic treatment with a fresh frozen plasma transfusion, 33% (5/15) of greyhounds experienced bleeding. After addition or substitution of this prophylactic treatment with EACA, the incidence of bleeding decreased to 16% (4/25) of greyhounds.

Despite these findings, the incidence of bleeding in dogs and the course of treatment - both prophylactically and therapeutically - remains erratic and unpredictable. This is especially evident given the unexplained occurrences in post-operative bleeding that occur several days after the surgical procedure takes place. Therefore, there exists a need for new methods to identify animals at risk for bleeding complications and treatment regimens to reduce the severity of the diagnosis. Accordingly, the present disclosure provides novel methods to identify animals via genetic testing and treat the animals at risk to prevent or reduce harmful bleeding episodes. In particular, the present disclosure provides determination of genetic mutations in the SERPINF2 gene and associated methods thereof.

The methods of the present disclosure provide several benefits compared to the current state of the art. The methods offer a simple genetic test to rapidly identify animals at risk for bleeding complications. Further, the methods provide a targeted treatment strategy for at-risk animals to mitigate a problem that, up to this point, has been unpredictable in nature. Further, identification of the described genetic mutation can also be beneficial in other methods as well, such as breeding decisions of animals to maximize the value of potential offspring.

Other objects, features and advantages of the present disclosure will become apparent from the following detailed description. It should be understood, however, that the detailed description and the specific examples, while indicating specific embodiments of the invention, are given by way of illustration only, since various changes and modifications within the spirit and scope of the invention will become apparent to those skilled in the art from this detailed description.

Lind B. and S. Thorsen, British Journal of Haematology (1999), 107: 317-322 reports a novel missense mutation in the human plasmin inhibitor (alpha₂-antiplasmin) gene associated with a bleeding tendency. Jain S. and S.S. Acharya, Transfusion and Apheresis Science (2019), 58: 572-577 reports on inherited disorders of the fibrinolytic pathway. Law R.H.P. et al, Blood (2008), 111(4): 2049-2052 reports an X-ray crystal structure of the fibrinolysis inhibitor α₂-antiplasmin. US2008/0057491 A1 reports inhibitors of anti-plasmin cleaving enzyme for use in various therapies related to fibrin and α₂-antiplasmin, and to substrates of APCE, which may be used, for example, in screening methods for identifying such inhibitors. Christiansen V.J. et al, Blood (2007), 109(12):5286-5292 reports on the effect of a single nucleotide polymorphism on human α₂-antiplasmin activity. WO 2013/156785 A1 relates to the use of the β-III spectrin gene (SPTBN2) as a biomarker for the in vitro diagnosis of cerebellar cortical degeneration, to in vitro methods of assessing the cerebellar cortical degeneration in a canine mammal and to primers and diagnostic kits for use in said method. EP 3124619 B1 relates to reagents, methods and kits for across and within dog breed glaucoma diagnosis. US 2015/148406 A1 relates to the use of the TTC8 gene as a biomarker for the prognosis of a canine mammal developing progressive retinal atrophy. EP 3412776 A1 relates to a genetic polymorphism associated with dog afibrinogenemia.

### BRIEF DESCRIPTION OF THE DRAWINGS

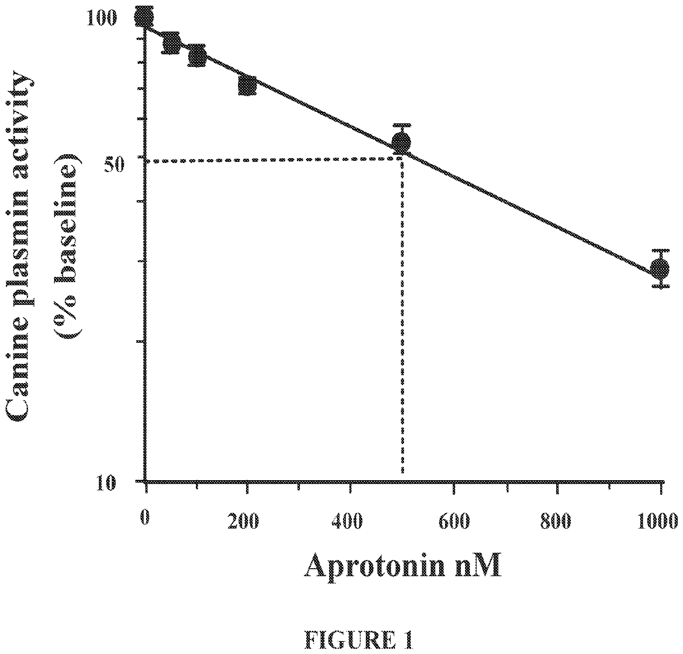

FIGURE 1 shows concentration-dependent inhibition of plasmin activity by the antifibrinolytic aprotinin (Cat.# A1153, Sigma Aldrich, St Louis, MO) with an IC50 of approximately 500 nM.

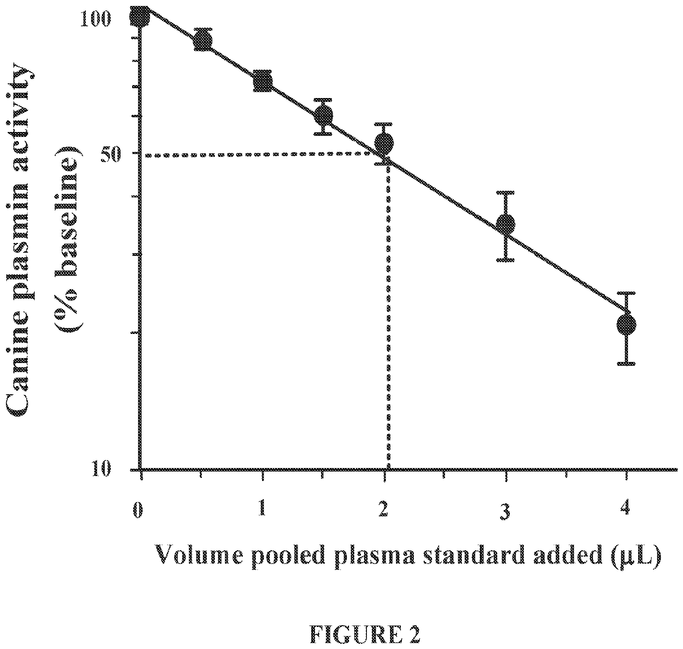

FIGURE 2 shows plots of plasma standard volume versus log-transformed percent plasmin activity were consistently linear (R² >0.99) over the assay range (0 µL to 4 µL).

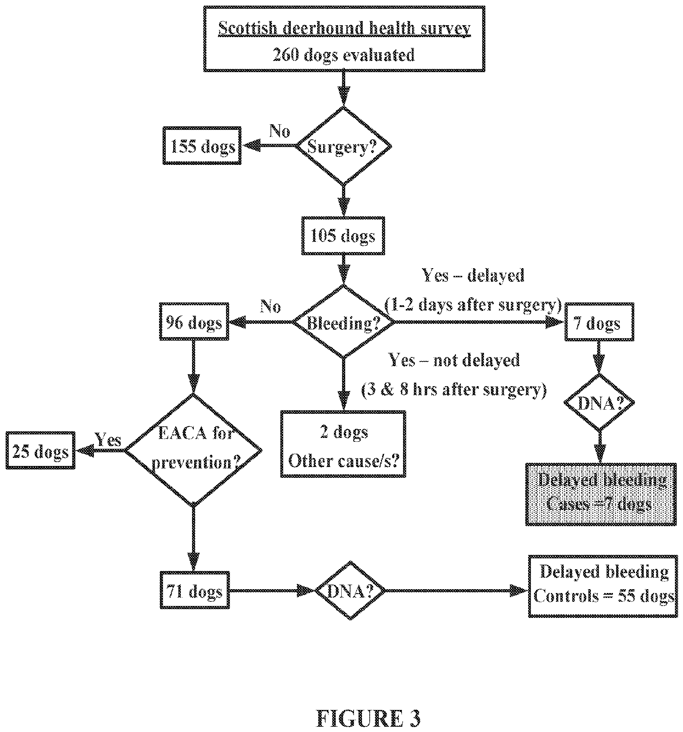

FIGURE 3 shows identification of delayed postoperative bleeding cases and controls. The flowchart shows the stepwise process used to identify delayed postoperative bleeding cases and controls for genotype-phenotype analysis. A study population of 260 Scottish deerhound dogs with completed health surveys was evaluated. Case inclusion criteria included a history of a surgical procedure with evidence for postoperative bleeding starting from 1 to 4 days after surgery. Cases were excluded if the bleeding occurred within 1 day or more than 4 days after surgery. Controls were included if they had a history of surgery, and excluded if they had received an antifibrinolytic drug (epsilon aminocaproic acid; EACA) before surgery. The final inclusion criteria for both cases and controls was the availability of DNA samples (from DNA repositories or directly from the dog) for genotyping.

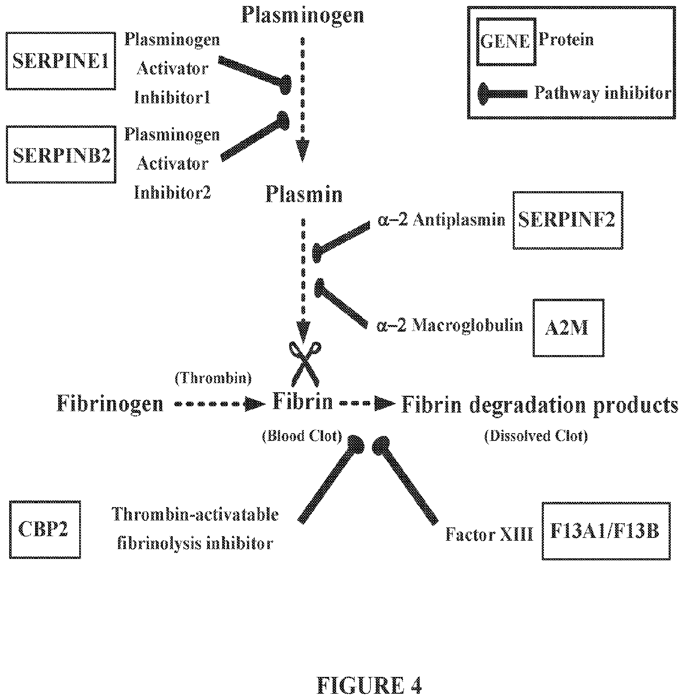

FIGURE 4 shows candidate genes (and associated protein products) involved in the negative regulation of plasmin-mediated degradation of fibrin. The coding regions of these genes were sequenced using DNA obtained from a discovery subset of 10 Scottish deerhound dogs (4 cases and 6 controls).

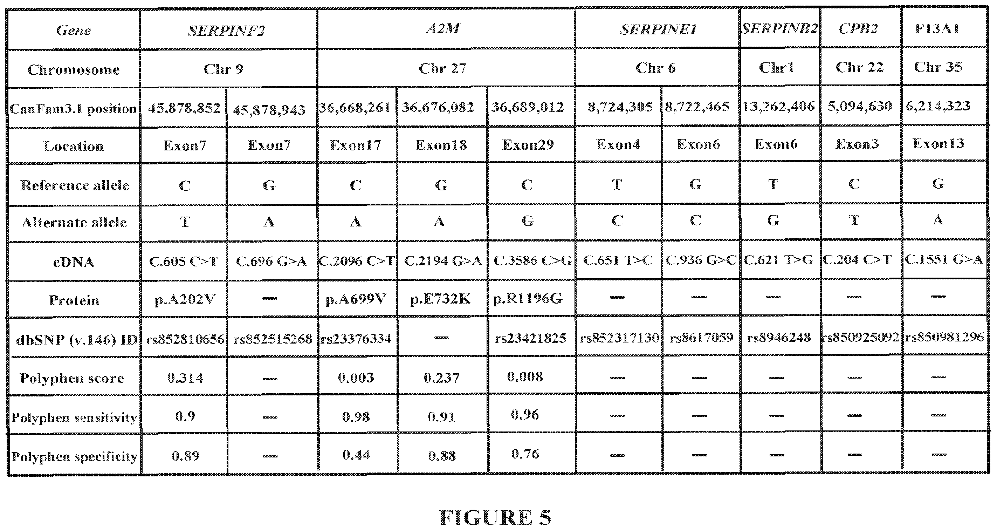

FIGURE 5 shows protein coding region gene variants identified by gene-capture sequencing of DNA samples obtained from 10 Scottish deerhound dogs (4 cases and 6 controls).

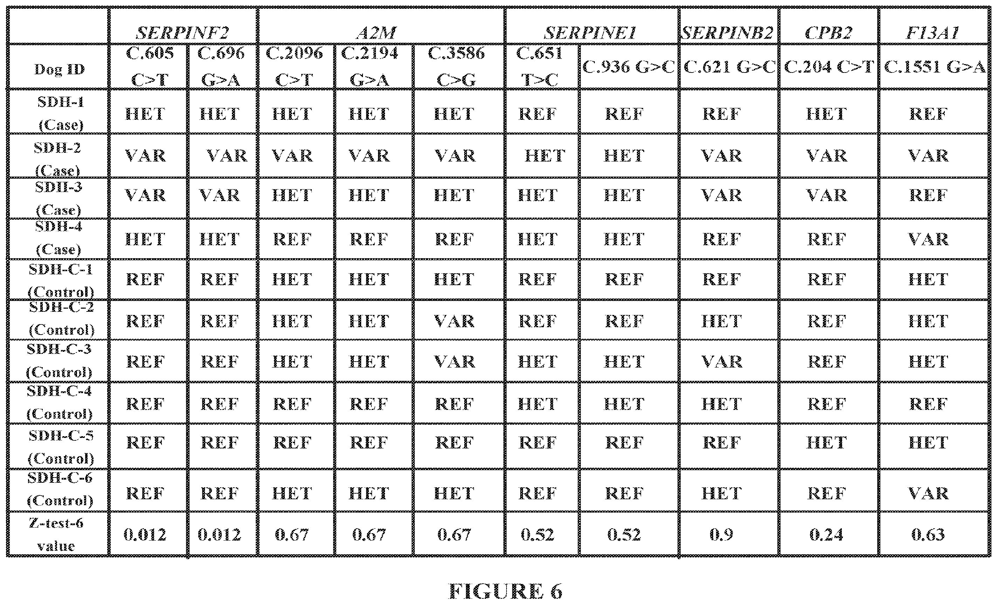

FIGURE 6 shows genotypes of candidate gene coding region variants identified by gene-capturing sequencing of DNA samples in a subset of 10 Scottish deerhound dogs with known postoperative bleeding phenotype (4 cases and 6 controls). Also shown are the results of a Z-test (P value) comparing the proportion of dogs with at least one variant allele in case and control groups. Genotypes are shown as either homozygous reference (REF), heterozygous (HET), or homozygous variant (VAR).

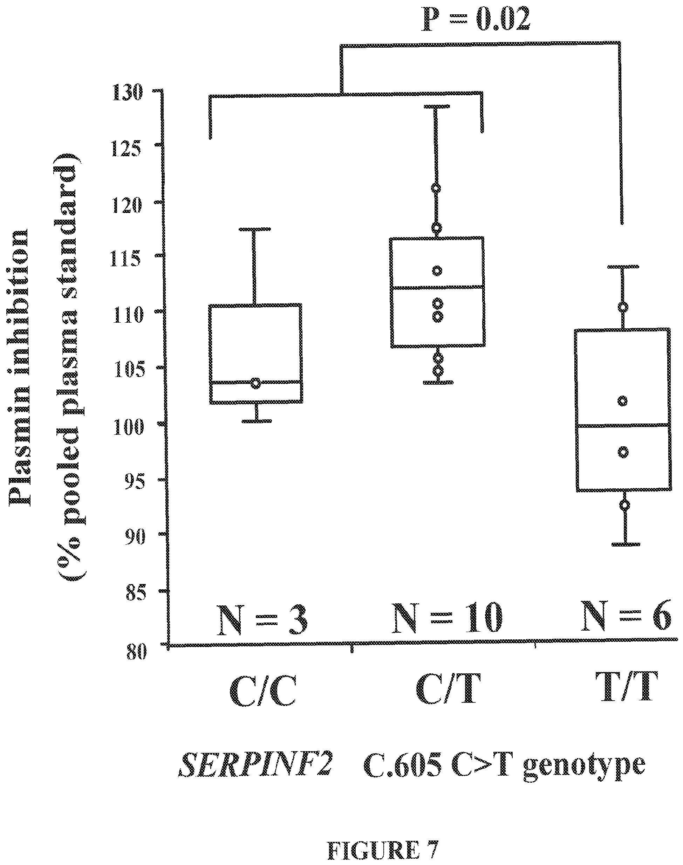

FIGURE 7 shows association of plasma antiplasmin activities with *SERPINF2* c.605 C>T genotype. Plasma was collected from 19 healthy greyhound dogs that were genotyped for the *SERPINF2* c.605 C>T mutation. Plasma samples were assayed for the ability to inhibit canine plasmin enzyme activity using a validated microplate-based colorimetric kinetic assay. Results were expressed relative to a pooled standard plasma sample, which was set as 100%. Shown are box and whiskers plots of plasmin inhibition data for each genotype, the numbers of samples with each genotype, and the results of comparison of activities between T/T (N = 6) and C/C combined with C/T (N = 13) groups (P value of Students t-test).

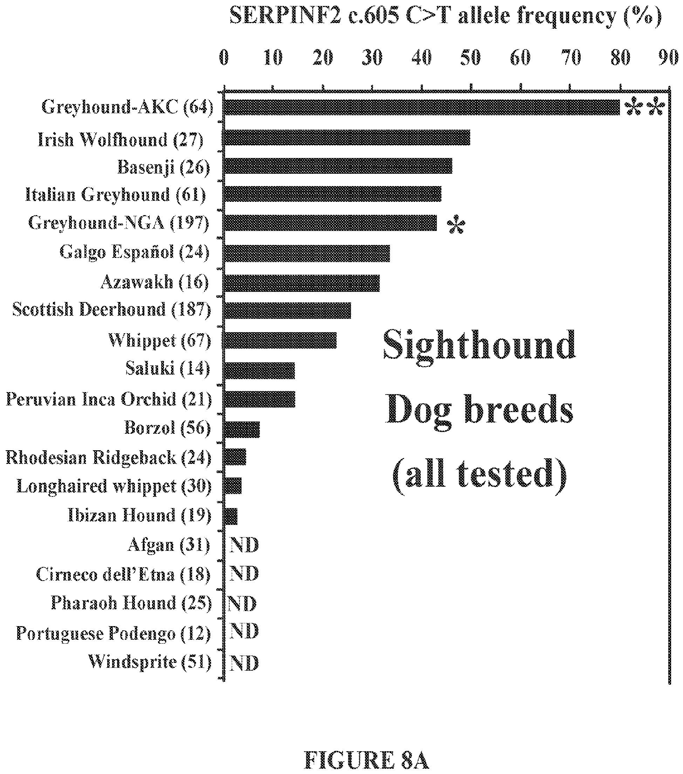

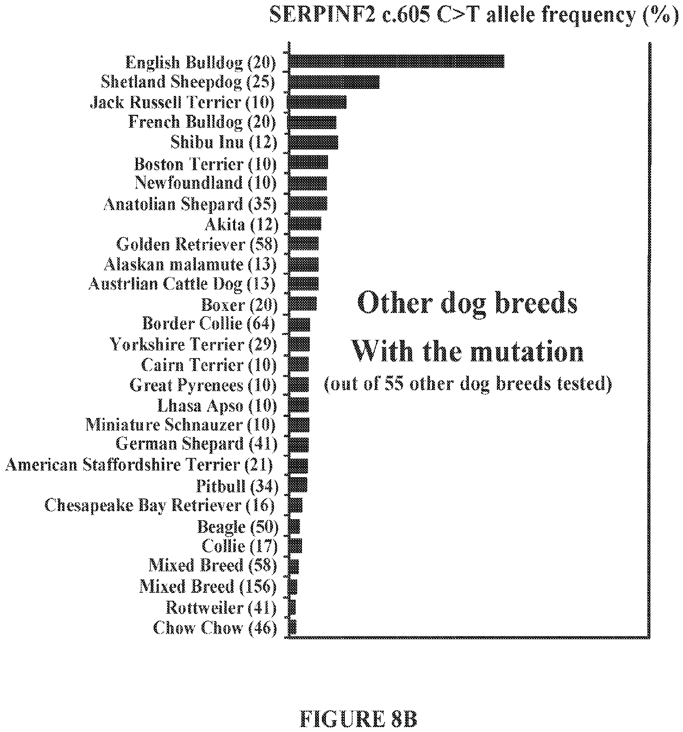

FIGURES 8A-8B show the heterogeneity of the SERPINF2 c.605 T allele across dog breeds. *SERPINF2* c.605 C>T allele frequencies were determined by genotyping 2,298 DNA samples collected from 75 different breeds, including 20 Sighthound breeds, 55 other (non-Sighthound) breeds, and 156 mixed-breed dogs. Breeds were designated by the dog's owner. Greyhounds were divided into two breed groups based on whether they were identified by their owners as dogs registered with the National Greyhound Association (NGA*) bred for racing or were dogs registered with the American Kennel Club (AKC**) bred for other purposes. Variant *SERPINF2* c.605 T allele frequencies are shown for individual breeds grouped into "Sighthound dog breeds" (i.e., FIG. 8A) and "Other dog breeds" i.e., FIG. 8B) for comparison. Shown next to the breed name are the number of individual dogs that were sampled. At least 10 dogs were sampled per breed. Dog breeds (N dogs) in which the mutation was tested but was not detected included Australian shepherd (15), basset hound (10), Bernese mountain dog (27), Brittany spaniel (25), Cardigan Welsh corgi (22), Chihuahua (14), cocker spaniel (26), dachshund (11), Doberman pinscher (35), German shorthaired pointer (11), great Dane (10), miniature dachshund (20), miniature poodle (10), Pembroke Welsh corgi (22), Pomeranian (10), pug (10), Saint Bernard (23), Samoyed (12), shar pei (10), shih tzu (10), Siberian husky (21), soft-coated Wheaten terrier (26), springer spaniel (11), standard poodle (21), Tibetan terrier (11), toy poodle (19), and Weimaraner (15). ND = *SERPINF2* c.605 T allele not detected.

### DETAILED DESCRIPTION

The following are definitions of terms that may be used in the present specification. The initial definition provided for a group or term herein applies to that group or term throughout the present specification individually or as part of another group, unless otherwise indicated. Additionally, it will be understood that any list of such candidates or alternatives is merely illustrative, not limiting, unless implicitly or explicitly understood or stated otherwise.

As used in this specification and the appended claims, the singular forms "a", "an" and "the" include plural referents unless the content clearly dictates otherwise. The use of the word "a" or "an" when used in conjunction with the term "comprising" in the claims and/or the specification may mean "one," but it is also consistent with the meaning of "one or more," "at least one," and "one or more than one."

Throughout this application, the term "about" is used to indicate that a value includes the standard deviation of error for the device or method being employed to determine the value. The use of the term "or" in the claims is used to mean "and/or" unless explicitly indicated to refer to alternatives only or the alternatives are mutually exclusive, although the disclosure supports a definition that refers to only alternatives and "and/or."

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

Abbreviations are used throughout this application, the following are abbreviations: EACA - epsilon aminocaproic acid; SNV - single nucleotide variant; AKC - American Kennel Club; NGA - National Greyhound Association; SDH - Scottish deerhound; PCR - polymerase chain reaction; O.R. - odds ratio; C.I. - confidence interval.

In addition, unless otherwise indicated, numbers expressing quantities of ingredients, constituents, reaction conditions and so forth used in the specification and claims are to be understood as being modified by the term "about." Accordingly, unless indicated to the contrary, the numerical parameters set forth in the specification and attached claims are approximations that may vary depending upon the desired properties sought to be obtained by the subject matter presented herein. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the subject matter presented herein are approximations, the numerical values set forth in the specific examples are reported as precisely as possible. Any numerical values, however, inherently contain certain errors necessarily resulting from the standard deviation found in their respective testing measurements.

The invention is defined by the appended claims.

In an illustrative aspect, which is not part of the invention, a method of treating bleeding in an animal provided. The method comprises the steps of determining if a genetic mutation in a SERPINF2 gene is present in the animal, and treating the animal with a veterinary composition if the genetic mutation in the SERPINF2 gene is present in the animal.

As used herein, a veterinary composition refers to any drug or formulation that can suitably be administered to an animal. For instance, veterinary compositions can include active ingredients approved for use in animals and/or active ingredients approved for human use and formulated or compounded for utilization in a non-human species. In some embodiments, the veterinary composition is obtained from a compounding pharmacy that formulates the veterinary composition for use in a non-human species using an FDA-approved drug or a base ingredient that is manufactured in an FDA-approved facility.

SERPINF2 (Serpin Family F Member 2) is known in the art as a protein coding gene. The gene encodes a member of the serpin family of serine protease inhibitors. The resultant protein is a major inhibitor of plasmin, which degrades fibrin and various other proteins. As described herein, genetic mutations of the SERPINF2 gene and/or its resultant protein can be utilized according to the present disclosure. As described herein, genetic mutations of the SERPINF2 gene and/or its resultant protein can be utilized according to the present disclosure.
The wild type sequence of the SERPINF2 gene is shown as SEQ ID NO:1: 
cctgcctcgcctgcccacccctcaccacagtgtttgtgccttgaactgaggaggcagcccctgagcccagaactcttatggtccaacaccc
cgagagaaggggtgagaataatctctgcatctcaggtctcccaggaaatgggaagacccaggcatcccaggaccctcacattctggagc
agagatgagatctgagcctgtgcccctactgcctcctcattatttgcaatagatgtaaatatgaccaataaatcctttggaagagccatggac
ctgagtgatgcttttctcgacgatggcatcagggtggggaagaatcgagactcagtagcttccagggcagtgaagacagagtgagaagt
agggcaggagagtgggagacagcctaatcgctgcttttgggcaccacagggtacagggcttctggatcaagcagcccccctcagagga
ggctcagccctggcgggatgttaaggaggcattgctcctgctgagggaggatgctgcctggtactgtgaccctctctcctctccccactcc
ttctagatccagtctaaaaggcagccaatcaggatcctggcttgtaaggtttgaggatgggcatcttgagctccccacataagctcctacag
cttccccttcctccccctcatgataggggagaggaaagaacattggaagtgagaagctggatgtgactcgagcctagctctgtccttgggc
acgtcacctaccctctctgactccagtaaagtagagataaaccccccccacctcatggggttgctggaaggcccctgtccttcctggatctg
tcgtacagatgaggacatcttccttaactttcccgaatctcctttctccaggcgtgggtgggacccaaatccacagggctccaggtggctat
ctcaggattagatgccctggtgggaatggggtttccggccccagtatcaatgtttaaccggatgtgctggtcaatatttaccagaagagaaa
TCTGAACAATCTGAACAGGGGTTGGCTGAGAGCAAAGGTCCTGGTGGGAGGGGAG
GGCTCAGATGGGCTGGCTGGAGCCTGAGCAAAGTGTGGGGAGCACCACAGTGGGG
CCAGAGGGACATTGTGTGCAGCAGCAAGGAGCCTACAGAGgtacgaggggaggggtggcttgg
cctgctctttgggccagataggaggtaagagctgcccacccggctgaaggggtctggggtgaatttattcagaaggcctctcatttcccag
 
cctcctggggtgatgaggagccaaggtgggggttctggcaggagtctctctgggacttgaaaaactggaggtcttttcttgtcaggaggat
tcaaaacccccattagagattagggccctggccaagggtccttgtgtgtgatggaggtggggccaggggtgatagaggatcccctggaa
ggactggccctgagatgggggcggggttggagtgtatgggacctgctctgaggccagactaagacctgcccatccccacctgctggct
gggcttcccggagccgacctggacagagtcgggcacaaggcctatgtttcccgtaagtccaaaggtggctctatccagaaccattcattg
ggctctgtggctagGTCAACAACAAGGCTCTCCGGCCCATCCCACCAAACCTGCCGAGACC
TCAGGGATTCACAGGCCTGTCTACCCTTGTGGTCAAGACCAGACCAAGAAAAGgtgg
gccattgggcagcaggttgggtaggagctctgggtaaggagccctaggacacaggaggcttcagggcaggcttcccagaagaggggc
cttgggctaggctgacaacagccataccactgagtgccaggcagcgtaaccagggttcacaaagactgtctcattttggtcctcacagtat
accgtgggaggaggtagggacttttgctaccattttacaagaaaaggcaaagcccagggagctatctgagggcctctgggaagaggca
gagtctggattccaatcgatgttggactcacaatgccaggcctcgaagaatgaggacttctttcattttgattatttcccctatgaaccaaaac
attttcagtgcatgacatgtggaaaactggaaaattcaaaagggctgaaaaggattcagggggatatcccgccccacggagatgagaaa
cagggctcgtgggtgttgggagtgctggcggtggtggctggattgacctgccctcccaggcagcgtgtgggtgggcccccgggagtgg
ggtcggtggggacggggacgcaggctgcctgtccccgcacagGAACATGGCGCTGCTCCGGGGGCTCCTG
GTGATCAGCTTGTCCTGCCTCCAAGGCTCCGGCGCGGTGgtgagcctgggcctccctggggggga
gggggctgagtcaagggagcgtttggctctgaagagctccctctcttcccctcatccttttctcgacagTTCCCTCCTGCCAGC
GCCATGGAGCCCTTGGACCAGCAGgtactcgggagtgagaggcaggagggggaggaaagccccgcgggccta
acctgtggttggcgcagggagtgggccgacccgtgggaagggtcacgctccatttgcttcctcccttcttcagCTAATGGGCGG
GCAGACCCAAGAGAAGCTGCCCCCGCTCACCCTCCTCAAGTTGGGCAACCAGgtacaa
ccaggtggggctggggaagagtgggcggggtccgagggaggagggccaatcagcggggccacgactcagagggtgggaggggc
cagaggaaaggaccaggtccttgtactgtcattgtccatgtgactgtccctctcttgccaggactattcttgggcaggggggctgtggaag
ggatcttcaacacagagcggactccttgaccctcttgacccctgatctgtctgtagGAGTCCAGTGGCCATATTGGCC
TGAAGAAGGCCCCAAGAGACTGCAGGGGAGCCCCAACCCCAGAGCAGACCCGCAG
ATTGGCCCAGGCCATGATGGCCTTCACCACAGACCTGTTCTCCCTGGTGGCCCAAA
GGTCCACCAGCCCCAACCTAATCCTGTCGCCTCTGAGTGTGGCCCTGGCACTGTCTC
ACCTGGCACTAGgtactgtggcatcacctgtccagaccagcagagctggaggccagtgggaactcactactccagagtttact
agtgggtgcctcctgcatcagagtcacttggatgtttggtaaaaatgcagattcctaggtacacctccagtctctgtgaatcagattcccagg
gattgggcctaaggatatgcttctggtgatttttacataagcctttgataactacaatgtctccaccggactggagactctcattgtgggaaac
cgaggtccagagagggaatacgacttggatcgagtgaggtgacccagactgccagcagcagagacaaaagtagaacttggggagtatt
gtcaccccaatcagtttagtggatacggggggagaatgtgtggtgtcgtggccatgctcacgatggggatttaacagagcctggagtggc
cagtaggggtgggaaagtgccactggcctgggccaaagcccatgtggtcccctccagGTGCTCAGAACCAGACGCT
GCGGAGGTTAGAGCAGGTGCTGCATGCAGACTCTGGGCCCTGCCTCCCCCACCTGC
TGAGCCGCCTTTGCCAGGACCTGGGCCCTGGGGCATTCCGATTGGCTGCTAGAATG
TACCTGCAGAAAGgtaggtgctggtggcttggagctccctaggttgtgccctggagtggacaggagtgatggagatgggttt
 
ggccttaggcagccagtcagagcattggtggctctggagctcaagcccttaggaacagcttgtggccttttctgtgtagGATTTCCC
ATCAAAGAGGACTTCCTGAAACAATCAGAACAGCTCTTTGGTGCCAAGCCCATGAG
CCTGACCGGAAGGAAAGGGGATGATCTGCTAAACATCAACCAATGGGTGAAGGAG
GCCACAGAGGGGAAGATTGAGGATTTCCTCTCAGAGCTGCCAGATGACACAGTGTT
GCTTCTGCTCAATGCCATCCACTTCCAGGgtgcgttccccctcttctctggccctctacccctgtggcccctattc
ctgccttgtaggctgaactgggctgcaaaacctttgttctgagggtctctctttctcctcagggctgggtcaggaggcagggagctttctagg
ttgtggcttgggggagggtaggaaacaggtaatggtgtgccagtgaggggcctcaggaactggatgagagaaccctaggaggaaactg
agtctgaaaggattgagggtggggttgggggtgctcctgggagcatgacaggacataggaagaatgcaagcccagatttcaaaaatgct
gcagggaagaaacctcagatcaactgggaatcaactggcacagtgccccttgggcttttgacttctactttttgtcccttgaatgggttctgg
gtgggatggagaagagactagtggtgtggaacccacccctgtcccagtctacgtgacccctgccctctgctggatatagGTTTCTG
GAGGAGCAAGTTTGATCCAAGCCTCACCCAGAGAGACAGTTTCCACCTGAACGAG
CAGTTCACGGTGCCAGTGGACATGATGCACGCCCGCACGTACCCCCTGCGCTGGTT
CCTGCTGGAGCAGCCTGAGATACAGgtcacccttgactgcccaggctgggcctggctgctgggaggtggggca
gggggggcaggctgtgggacaggtacaggggtggaggggggtgtgccccacctcccttgcagtcttgtccctgagacaggtggctctg
gaaggtcacattatgccctgatccagagggactgaggttccaggctctttgaagttcaagaatttgacaaaactatattaggcatttgccacc
agaacacactagcacaagggagaccaagtccctgctctcaggaggaaagacacacacagtacacggacagacataaatcatggaataa
tttcacatagtataactgctatgaaggaaaaaaataaagtgatcaattagtgacaggggaaggtattttattttatttatttttttaaagaagttcttt
ttttaattttttttatttatttatgatagtcacagagagagagagagagagagagagagagaggcagagacataggcagagggaaaagcag
gctccatgcaccgggagcccgacgtgggatttgatcccaggtctcgaggatcacgccctgggccaaaggcaggcactaaaccgctgtg
ccacccagggatccctggggaaggtattttagataaagtggtcagggagcagtctatgaggaggggcctcaagtggagaaccagatgat
ttttggtcagaggggtttattacaagggcccatggattggtcaggctgcaaaggagccacccaacagctacagagccactcaggcctcag
catcctcgagcctgggagacgtgatctgactgtcacagtgctccttggaagggaggatctggttgcaatggatttgtcagtaacctgctgga
acattctgattggatagagtactatgccaagcggtctcaggtgaggcctcccttgcttctagcctaaatgtggtcctcttggcttgagcatgga
cccctgggcccatccactgtggcagagatggcaatgttggttagtttcccaaggcagcatgccacaggcctcagcgactcctgagaggg
gctgtgggggcaaggcagacccttggagcagcgtccagcttctctcgctttcaccccgacgggcagactgtcttgttcacctccccacctg
ttctctttgtgctgctcctgccctccaggctgtgtcctgcccactctttcttcacccatcctctctgtccctggcctctgccctcctcttcctcaca
cactctgcaggctgacccagacagccagatgtggtgttccccaccacccccgccacccccaaggttcctgctccctggctgctctgccta
gagactaagaagtctcacttgccagggatcatcatgcccagtgcttctcatactcaagtagctgcagaaaccccattcacatgacatcttac
ctcagatcacagtatcatatacaccctaggtgaaggtgtgggggggagaaccacctgttgcagtggctcctctgtaggacctagggtggtt
tgaaaacgaacaacccagtccattgtacacgtggagacactgagaggcagagctggttacagacgaagagagggaaagtccccccttt
attgagcactgcccatgtgctgatactccactagaagctttaaaagggtctctctctctcttttttttctctctctcacatgtcaacctacaatgtg
ggcaggcagtgttactccacttcacaggtgaggaagtgggggctcagggaggctgaacacacacctgagaacaatggactggaactcc
tgctccatctgtctcagaagcctatgctggcccagaccccagctggtatgggcaccttctggccctcaccgctgtctcctttctttctctgtag
 
GTGGCTCATTTCCCCTTTAAGAACAACATGAGCTTTGTGGTCATTATGCCCACCCAC
TTTGAGTGGAACGTGTCCCAGGTGCTGGCCAACCTGAGCTGGGACATCCTGCACCA
GCCCTTACTGCGAGAGAAACCCACCAAGGTCCGGCTGCCTAAGCTGCACCTGAACT
ACCAGCTGGACTTGGTGGCCACCCTCAGCCGGCTGGgtaaggaagggtgaggcttgggtaaggacag
tcccctaggccaggctaggcagagcaagtgaggaacaaagccccagctggcagtaggtccgagtcagagcagccttgtgtcctggcct
tctctggggctttgggaggtgagggcttcctaggaccccccagatcctccgtcccgggatgggctggggtggcccagggaatgaggatc
tgctccatccaagactgaacattgtgaagaatcttgggtagagagcctgcgtggcacgtgagccttgctggctgtcctgcaaggggcact
gtactaggtacaaggggctcaggcagatgcggaatcaggtacttactatacagcttaggtgatgagtcagagggcttctctccagttcact
ggagaagggcacccggccggaccttggggcaggggatccagaaagtctccttggaggaagtgacctttaagccaataactggaggtta
agtgagagttccccaaggatctgtgggttggaggtgttcccggccagaatgctagccaccgtcccaggcaggtcccggcagcctgcgg
ggcagaaggcagctctgaccggcagtctcttgccctgggcagGCCTGCAGGAGCTGTTCCAGGCCCCAGAC
CTGCGCGGGATCTCCGACCAGCGCCTGGTGGTGACCAGCGTGCAGCACCAGTCTGC
TCTGGAGCTCAGTGAGGCTGGTGTGGAGGCGGCCGCGGCCACGGGCACGGCCATG
TCCCGAATGTCTCTCTCCTCCTTCAGCGTCAACCGCCCCTTCCTCTTCTTCATCCTCG
AGGACACAACAAGCCTGCCCCTGTTTGTGGGCAGTGTGAAGAACCCCAACCCTAGT
GCGCCATGGGAGCGCAAGGAGCAGCAGGATTCCCCCGATGACAGGGACTACTTCC
AGAACCGGAAAGCCTTTCCCCGCGGAGACAAGCCCTTCGGGCCTGACTTGAAACTT
GCGCCTCCCTCAGAGGAGGATTACCCCCAACTTCATAGCCCCAAGTGAGAGGCCAG
CTGCCAGCATCCCGAGTCCCTGCTGGAGCAACCTCTCAACTCTTGTGACTCTTTCCA
GACAGCATTGTGGGAGGAGGGCAGGGACCTGGGTGGGGAGTCTGGGAGCCCAGAG
GGAGCCACTCTCTCAAGTCTCCTCCTCTCAGGGATTTTGGGGTTGGGGGGGCCACA
GGACGGGGCGGACCTCAGGTAATCAAGGGCCTTCATCCCACATCTCTTCTTCCAAA
AGCGACTCAGAGGCTGTCCTTTGGGGGACTTGGGCAGAAGGGCAGCTGGAGAAGC
TGGAGATCCACTTTTGTCAGGAAGGTAGTTCCCGTGTTGGCTGTGGCACTTCCACGT
TTGTTTACGGGGGGGGGGGGGTGTTGGGGGGGCAGGGGGGCAGGAGGGATGGAAT
ACCTTGGCTGGGGAGAGGACAGTAGGCCCTGGGGGTGTGGAGCCTGGGCCTGCTCT
ATGGGCGGGGCCTAGAATTCCACAAACTGCTTCTGCCACACTTGCTGGAGGCCCTC
CAGCCCCCATCCCCCAACCCGCCCCCCACACCCTGTCACTTCAGCCTCCCCACTGCT
CCTAGGAGATACCAGCATTGCCAGGACTCTTCTTCCCTGTCTCTCCTCTTCTTCCTCT
GCCCAGGTGTCTCAGGTGGGAGGTGTGTGTGTGGGGAATAGTTAACTCCCTAAGGC
TCTTTTGTAAAGTTTTTGTAGTGATTTTTATGCCACCTGAATAAAGAATGAATGACC
CTGGCTGTTTTGATGTCACTCTGCTGGGCATGGGCTGGAGCAAGGGGGCTGAGGGA
GGCAGGAGGGCCCTGGCGGCACTGAGCCTCACGCTGGGCCCTGACAGCACACAGC
 
AGGCGGccctctctgccacgtttgtcccctggccacccgccagctcttccccggcgcagtcccctctgaccttgccagccctctctgc
ggccagctccatgccagccgggctccactctctccagaggcctcaagcagccattaacaagggtggctcagaatagcgctggcctccca
gtttggagaaaactggcaattagggagcttgtggagtttctaattacatgtcagtccttctggcaggggcaggcgcccgtcagccagggac
aggctgcagggagcactggttccagctggacgctgtcatgcctgaggatctgggggaaacagttgggcccaccttggcccggggccgt
gggtggtatgcagggccacgtgcgtgggcccttatctgtgggtacagggtgtccctgtggccgtggccacctgaagccccgaagtgcag
tggtcaagagcaagtgcccagggtggactctggcttcctacaaaccagcaatgtggggagacaggtgaaatcgatgatgaggcagagg
gtggttggttagagccgtggtgagaaatagagcagggaaggcctccctgagaaggtggtacgggagcagagacttgaggcaggtgaa
ggcaggcatgatataaataaccagggcagagttcctgggtggaagcatgctcagaaggttcaggatggggatccctgggtggcgcagc
ggtttggcgcttgcctttggcccagggcgcgatcctggagacccgggatcaaatcccacgtcgggctcctggtgcatggagcccgcttct
ccctcaacctgtgtctctgcctctctctctctctctgtgactatcataaaaaaaaaaaaaaattagaaggttcaggatgaacagggggtccgt
ggagctagaggggaggggtaggagtgagtcagaggggaacatgtactagtgctttagggccactgtaaagactgttgtttttatcttagga
gtaagaagtaattggatagttaaa (SEQ ID NO:1)
The cDNA sequence of the SERPINF2 gene is shown as SEQ ID NO:2: 
atgggctggctggagcctgagcaaagtgtggggagcaccacagtggggccagagggacattgtgtgcagcagcaaggagcctacag
aggtcaacaacaaggctctccggcccatcccaccaaacctgccgagacctcagggattcacaggcctgtctacccttgtggtcaagacc
agaccaagaaaaggaacatggcgctgctccgggggctcctggtgatcagcttgtcctgcctccaaggctccggcgcggtgttccctcct
gccagcgccatggagcccttggaccagcagctaatgggcgggcagacccaagagaagctgcccccgctcaccctcctcaagttgggc
aaccaggagtccagtggccatattggcctgaagaaggccccaagagactgcaggggagccccaaccccagagcagacccgcagatt
ggcccaggccatgatggccttcaccacagacctgttctccctggtggcccaaaggtccaccagccccaacctaatcctgtcgcctctgag
tgtggccctggcactgtctcacctggcactaggtgctcagaaccagacgctgcggaggttagagcaggtgctgcatgcagactctgggc
cctgcctcccccacctgctgagccgcctttgccaggacctgggccctggggcattccgattggctgctagaatgtacctgcagaaaggatt
tcccatcaaagaggacttcctgaaacaatcagaacagctctttggtgccaagcccatgagcctgaccggaaggaaaggggatgatctgct
aaacatcaaccaatgggtgaaggaggccacagaggggaagattgaggatttcctctcagagctgccagatgacacagtgttgcttctgct
caatgccatccacttccagggtttctggaggagcaagtttgatccaagcctcacccagagagacagtttccacctgaacgagcagttcacg
gtgccagtggacatgatgcacgcccgcacgtaccccctgcgctggttcctgctggagcagcctgagatacaggtggctcatttccccttta
agaacaacatgagctttgtggtcattatgcccacccactttgagtggaacgtgtcccaggtgctggccaacctgagctgggacatcctgca
ccagcccttactgcgagagaaacccaccaaggtccggctgcctaagctgcacctgaactaccagctggacttggtggccaccctcagcc
ggctgggcctgcaggagctgttccaggccccagacctgcgcgggatctccgaccagcgcctggtggtgaccagcgtgcagcaccagt
ctgctctggagctcagtgaggctggtgtggaggcggccgcggccacgggcacggccatgtcccgaatgtctctctcctccttcagcgtca
accgccccttcctcttcttcatcctcgaggacacaacaagcctgcccctgtttgtgggcagtgtgaagaaccccaaccctagtgcgccatg
 
ggagcgcaaggagcagcaggattcccccgatgacagggactacttccagaaccggaaagcctttccccgcggagacaagcccttcgg
gcctgacttgaaacttgcgcctccctcagaggaggattacccccaacttcatagccccaagtga (SEQ ID NO:2)
The amino acid sequence of the SERPINF2 gene is shown as SEQ ID NO:3:

MGWLEPEQSVGSTTVGPEGHCVQQQGAYRGQQQGSPAHPTKPAETSGIHRPVYPCGQ
DQTKKRNMALLRGLLVISLSCLQGSGAVFPPASAMEPLDQQLMGGQTQEKLPPLTLLK
LGNQESSGHIGLKKAPRDCRGAPTPEQTRRLAQAMMAFTTDLFSLVAQRSTSPNLILSP
LSVALALSHLALGAQNQTLRRLEQVLHADSGPCLPHLLSRLCQDLGPGAFRLAARMYL
QKGFPIKEDFLKQSEQLFGAKPMSLTGRKGDDLLNINQWVKEATEGKIEDFLSELPDDT
VLLLLNAIHFQGFWRSKFDPSLTQRDSFHLNEQFTVPVDMMHARTYPLRWFLLEQPEI
QVAHFPFKNNMSFVVIMPTHFEWNVSQVLANLSWDILHQPLLREKPTKVRLPKLHLNY
QLDLVATLSRLGLQELFQAPDLRGISDQRLVVTSVQHQSALELSEAGVEAAAATGTAM
SRMSLSSFSVNRPFLFFILEDTTSLPLFVGSVKNPNPSAPWERKEQQDSPDDRDYFQNRK
AFPRGDKPFGPDLKLAPPSEEDYPQLHSPK (SEQ ID NO:3)

**In** an embodiment, the step of treating is a prophylactic treatment. Generally, a prophylactic treatment can refer to treating an animal before a surgical procedure. For instance, a prophylactic treatment can refer to treating the animal at any point up to about 1 day prior to a surgical procedure, including immediately before such procedure.

**In** an embodiment, the step of treating is given before a trauma inducing incident of the animal. **In** an embodiment, the step of treating is given after a trauma inducing incident of the animal. For instance, a trauma inducing incident can refer to an injury to the animal or a surgical procedure. **In** an embodiment, the step of treating is given before whelping of the animal. **In** an embodiment, the step of treating is given after whelping of the animal.

In an embodiment, the bleeding in the animal is delayed bleeding. As used herein, a delayed bleeding can refer to bleeding from the animal that is evident at any time within 5 days after a surgical procedure. For instance, delayed bleeding can be evident within 6 hours after a surgical procedure, or within 8 hours after a surgical procedure, or within 12 hours after a surgical procedure, or within 1 day after a surgical procedure, or within 2 days after a surgical procedure, or within 3 days after a surgical procedure, or within 4 days after a surgical procedure, or within 5 days after a surgical procedure. In an embodiment, the delayed bleeding is associated with a surgery performed on the animal. In an embodiment, the bleeding in the animal is subsequent to a surgical procedure.

In an embodiment, the genetic mutation indicates that the animal is sensitive to experience delayed bleeding, wherein the genetic mutation is a single nucleotide variant, wherein the single nucleotide variant is a non-synonymous gene mutation, wherein the genetic mutation in the SERPINF2 gene is c.605 C>T (i.e., a cytosine → thymine mutation at nucleotide position 605, wherein the genetic mutation in the SERPINF2 gene is p.A202V (i.e., an alanine → valine mutation at amino acid position 202).
A sequence of the SERPINF2 gene containing a mutation of interest (c.605 C>T) is shown as SEQ ID NO:4: 
cctgcctcgcctgcccacccctcaccacagtgtttgtgccttgaactgaggaggcagcccctgagcccagaactcttatggtccaacaccc
cgagagaaggggtgagaataatctctgcatctcaggtctcccaggaaatgggaagacccaggcatcccaggaccctcacattctggagc
agagatgagatctgagcctgtgcccctactgcctcctcattatttgcaatagatgtaaatatgaccaataaatcctttggaagagccatggac
ctgagtgatgcttttctcgacgatggcatcagggtggggaagaatcgagactcagtagcttccagggcagtgaagacagagtgagaagt
agggcaggagagtgggagacagcctaatcgctgcttttgggcaccacagggtacagggcttctggatcaagcagcccccctcagagga
ggctcagccctggcgggatgttaaggaggcattgctcctgctgagggaggatgctgcctggtactgtgaccctctctcctctccccactcc
ttctagatccagtctaaaaggcagccaatcaggatcctggcttgtaaggtttgaggatgggcatcttgagctccccacataagctcctacag
cttccccttcctccccctcatgataggggagaggaaagaacattggaagtgagaagctggatgtgactcgagcctagctctgtccttgggc
acgtcacctaccctctctgactccagtaaagtagagataaaccccccccacctcatggggttgctggaaggcccctgtccttcctggatctg
tcgtacagatgaggacatcttccttaactttcccgaatctcctttctccaggcgtgggtgggacccaaatccacagggctccaggtggctat
ctcaggattagatgccctggtgggaatggggtttccggccccagtatcaatgtttaaccggatgtgctggtcaatatttaccagaagagaaa
TCTGAACAATCTGAACAGGGGTTGGCTGAGAGCAAAGGTCCTGGTGGGAGGGGAG
GGCTCAGATGGGCTGGCTGGAGCCTGAGCAAAGTGTGGGGAGCACCACAGTGGGG
CCAGAGGGACATTGTGTGCAGCAGCAAGGAGCCTACAGAGgtacgaggggaggggtggcttgg
cctgctctttgggccagataggaggtaagagctgcccacccggctgaaggggtctggggtgaatttattcagaaggcctctcatttcccag
cctcctggggtgatgaggagccaaggtgggggttctggcaggagtctctctgggacttgaaaaactggaggtcttttcttgtcaggaggat
tcaaaacccccattagagattagggccctggccaagggtccttgtgtgtgatggaggtggggccaggggtgatagaggatcccctggaa
ggactggccctgagatgggggcggggttggagtgtatgggacctgctctgaggccagactaagacctgcccatccccacctgctggct
gggcttcccggagccgacctggacagagtcgggcacaaggcctatgtttcccgtaagtccaaaggtggctctatccagaaccattcattg
ggctctgtggctagGTCAACAACAAGGCTCTCCGGCCCATCCCACCAAACCTGCCGAGACC
TCAGGGATTCACAGGCCTGTCTACCCTTGTGGTCAAGACCAGACCAAGAAAAGgtgg
gccattgggcagcaggttgggtaggagctctgggtaaggagccctaggacacaggaggcttcagggcaggcttcccagaagaggggc
 
cttgggctaggctgacaacagccataccactgagtgccaggcagcgtaaccagggttcacaaagactgtctcattttggtcctcacagtat
accgtgggaggaggtagggacttttgctaccattttacaagaaaaggcaaagcccagggagctatctgagggcctctgggaagaggca
gagtctggattccaatcgatgttggactcacaatgccaggcctcgaagaatgaggacttctttcattttgattatttcccctatgaaccaaaac
attttcagtgcatgacatgtggaaaactggaaaattcaaaagggctgaaaaggattcagggggatatcccgccccacggagatgagaaa
cagggctcgtgggtgttgggagtgctggcggtggtggctggattgacctgccctcccaggcagcgtgtgggtgggcccccgggagtgg
ggtcggtggggacggggacgcaggctgcctgtccccgcacagGAACATGGCGCTGCTCCGGGGGCTCCTG
GTGATCAGCTTGTCCTGCCTCCAAGGCTCCGGCGCGGTGgtgagcctgggcctccctggggggga
gggggctgagtcaagggagcgtttggctctgaagagctccctctcttcccctcatccttttctcgacagTTCCCTCCTGCCAGC
GCCATGGAGCCCTTGGACCAGCAGgtactcgggagtgagaggcaggagggggaggaaagccccgcgggccta
acctgtggttggcgcagggagtgggccgacccgtgggaagggtcacgctccatttgcttcctcccttcttcagCTAATGGGCGG
GCAGACCCAAGAGAAGCTGCCCCCGCTCACCCTCCTCAAGTTGGGCAACCAGgtacaa
ccaggtggggctggggaagagtgggcggggtccgagggaggagggccaatcagcggggccacgactcagagggtgggaggggc
cagaggaaaggaccaggtccttgtactgtcattgtccatgtgactgtccctctcttgccaggactattcttgggcaggggggctgtggaag
ggatcttcaacacagagcggactccttgaccctcttgacccctgatctgtctgtagGAGTCCAGTGGCCATATTGGCC
TGAAGAAGGCCCCAAGAGACTGCAGGGGAGCCCCAACCCCAGAGCAGACCCGCAG
ATTGGCCCAGGCCATGATGGCCTTCACCACAGACCTGTTCTCCCTGGTGGCCCAAA
GGTCCACCAGCCCCAACCTAATCCTGTCGCCTCTGAGTGTGGCCCTGGCACTGTCTC
ACCTGGCACTAGgtactgtggcatcacctgtccagaccagcagagctggaggccagtgggaactcactactccagagtttact
agtgggtgcctcctgcatcagagtcacttggatgtttggtaaaaatgcagattcctaggtacacctccagtctctgtgaatcagattcccagg
gattgggcctaaggatatgcttctggtgatttttacataagcctttgataactacaatgtctccaccggactggagactctcattgtgggaaac
cgaggtccagagagggaatacgacttggatcgagtgaggtgacccagactgccagcagcagagacaaaagtagaacttggggagtatt
gtcaccccaatcagtttagtggatacggggggagaatgtgtggtgtcgtggccatgctcacgatggggatttaacagagcctggagtggc
cagtaggggtgggaaagtgccactggcctgggccaaagcccatgtggtcccctccagGTGCTCAGAACCAGACGCT
GCGGAGGTTAGAGCAGGTGCTGCATGTAGACTCTGGGCCCTGCCTCCCCCACCTGC
TGAGCCGCCTTTGCCAGGACCTGGGCCCTGGGGCATTCCGATTGGCTGCTAGAATG
TACCTGCAGAAAGgtaggtgctggtggcttggagctccctaggttgtgccctggagtggacaggagtgatggagatgggttt
ggccttaggcagccagtcagagcattggtggctctggagctcaagcccttaggaacagcttgtggccttttctgtgtagGATTTCCC
ATCAAAGAGGACTTCCTGAAACAATCAGAACAGCTCTTTGGTGCCAAGCCCATGAG
CCTGACCGGAAGGAAAGGGGATGATCTGCTAAACATCAACCAATGGGTGAAGGAG
GCCACAGAGGGGAAGATTGAGGATTTCCTCTCAGAGCTGCCAGATGACACAGTGTT
GCTTCTGCTCAATGCCATCCACTTCCAGGgtgcgttccccctcttctctggccctctacccctgtggcccctattc
ctgccttgtaggctgaactgggctgcaaaacctttgttctgagggtctctctttctcctcagggctgggtcaggaggcagggagctttctagg
ttgtggcttgggggagggtaggaaacaggtaatggtgtgccagtgaggggcctcaggaactggatgagagaaccctaggaggaaactg
 
agtctgaaaggattgagggtggggttgggggtgctcctgggagcatgacaggacataggaagaatgcaagcccagatttcaaaaatgct
gcagggaagaaacctcagatcaactgggaatcaactggcacagtgccccttgggcttttgacttctactttttgtcccttgaatgggttctgg
gtgggatggagaagagactagtggtgtggaacccacccctgtcccagtctacgtgacccctgccctctgctggatatagGTTTCTG
GAGGAGCAAGTTTGATCCAAGCCTCACCCAGAGAGACAGTTTCCACCTGAACGAG
CAGTTCACGGTGCCAGTGGACATGATGCACGCCCGCACGTACCCCCTGCGCTGGTT
CCTGCTGGAGCAGCCTGAGATACAGgtcacccttgactgcccaggctgggcctggctgctgggaggtggggca
gggggggcaggctgtgggacaggtacaggggtggaggggggtgtgccccacctcccttgcagtcttgtccctgagacaggtggctctg
gaaggtcacattatgccctgatccagagggactgaggttccaggctctttgaagttcaagaatttgacaaaactatattaggcatttgccacc
agaacacactagcacaagggagaccaagtccctgctctcaggaggaaagacacacacagtacacggacagacataaatcatggaataa
tttcacatagtataactgctatgaaggaaaaaaataaagtgatcaattagtgacaggggaaggtattttattttatttatttttttaaagaagttcttt
ttttaattttttttatttatttatgatagtcacagagagagagagagagagagagagagagaggcagagacataggcagagggaaaagcag
gctccatgcaccgggagcccgacgtgggatttgatcccaggtctcgaggatcacgccctgggccaaaggcaggcactaaaccgctgtg
ccacccagggatccctggggaaggtattttagataaagtggtcagggagcagtctatgaggaggggcctcaagtggagaaccagatgatt
tttggtcagaggggtttattacaagggcccatggattggtcaggctgcaaaggagccacccaacagctacagagccactcaggcctcag
catcctcgagcctgggagacgtgatctgactgtcacagtgctccttggaagggaggatctggttgcaatggatttgtcagtaacctgctgga
acattctgattggatagagtactatgccaagcggtctcaggtgaggcctcccttgcttctagcctaaatgtggtcctcttggcttgagcatgga
cccctgggcccatccactgtggcagagatggcaatgttggttagtttcccaaggcagcatgccacaggcctcagcgactcctgagaggg
gctgtgggggcaaggcagacccttggagcagcgtccagcttctctcgctttcaccccgacgggcagactgtcttgttcacctccccacctg
ttctctttgtgctgctcctgccctccaggctgtgtcctgcccactctttcttcacccatcctctctgtccctggcctctgccctcctcttcctcaca
cactctgcaggctgacccagacagccagatgtggtgttccccaccacccccgccacccccaaggttcctgctccctggctgctctgccta
gagactaagaagtctcacttgccagggatcatcatgcccagtgcttctcatactcaagtagctgcagaaaccccattcacatgacatcttac
ctcagatcacagtatcatatacaccctaggtgaaggtgtgggggggagaaccacctgttgcagtggctcctctgtaggacctagggtggtt
tgaaaacgaacaacccagtccattgtacacgtggagacactgagaggcagagctggttacagacgaagagagggaaagtccccccttt
attgagcactgcccatgtgctgatactccactagaagctttaaaagggtctctctctctcttttttttctctctctcacatgtcaacctacaatgtg
ggcaggcagtgttactccacttcacaggtgaggaagtgggggctcagggaggctgaacacacacctgagaacaatggactggaactcc
tgctccatctgtctcagaagcctatgctggcccagaccccagctggtatgggcaccttctggccctcaccgctgtctcctttctttctctgtag
GTGGCTCATTTCCCCTTTAAGAACAACATGAGCTTTGTGGTCATTATGCCCACCCAC
TTTGAGTGGAACGTGTCCCAGGTGCTGGCCAACCTGAGCTGGGACATCCTGCACCA
GCCCTTACTGCGAGAGAAACCCACCAAGGTCCGGCTGCCTAAGCTGCACCTGAACT
ACCAGCTGGACTTGGTGGCCACCCTCAGCCGGCTGGgtaaggaagggtgaggcttgggtaaggacag
tcccctaggccaggctaggcagagcaagtgaggaacaaagccccagctggcagtaggtccgagtcagagcagccttgtgtcctggcct
tctctggggctttgggaggtgagggcttcctaggaccccccagatcctccgtcccgggatgggctggggtggcccagggaatgaggatc
tgctccatccaagactgaacattgtgaagaatcttgggtagagagcctgcgtggcacgtgagccttgctggctgtcctgcaaggggcact
 
gtactaggtacaaggggctcaggcagatgcggaatcaggtacttactatacagcttaggtgatgagtcagagggcttctctccagttcact
ggagaagggcacccggccggaccttggggcaggggatccagaaagtctccttggaggaagtgacctttaagccaataactggaggtta
agtgagagttccccaaggatctgtgggttggaggtgttcccggccagaatgctagccaccgtcccaggcaggtcccggcagcctgcgg
ggcagaaggcagctctgaccggcagtctcttgccctgggcagGCCTGCAGGAGCTGTTCCAGGCCCCAGAC
CTGCGCGGGATCTCCGACCAGCGCCTGGTGGTGACCAGCGTGCAGCACCAGTCTGC
TCTGGAGCTCAGTGAGGCTGGTGTGGAGGCGGCCGCGGCCACGGGCACGGCCATG
TCCCGAATGTCTCTCTCCTCCTTCAGCGTCAACCGCCCCTTCCTCTTCTTCATCCTCG
AGGACACAACAAGCCTGCCCCTGTTTGTGGGCAGTGTGAAGAACCCCAACCCTAGT
GCGCCATGGGAGCGCAAGGAGCAGCAGGATTCCCCCGATGACAGGGACTACTTCC
AGAACCGGAAAGCCTTTCCCCGCGGAGACAAGCCCTTCGGGCCTGACTTGAAACTT
GCGCCTCCCTCAGAGGAGGATTACCCCCAACTTCATAGCCCCAAGTGAGAGGCCAG
CTGCCAGCATCCCGAGTCCCTGCTGGAGCAACCTCTCAACTCTTGTGACTCTTTCCA
GACAGCATTGTGGGAGGAGGGCAGGGACCTGGGTGGGGAGTCTGGGAGCCCAGAG
GGAGCCACTCTCTCAAGTCTCCTCCTCTCAGGGATTTTGGGGTTGGGGGGGCCACA
GGACGGGGCGGACCTCAGGTAATCAAGGGCCTTCATCCCACATCTCTTCTTCCAAA
AGCGACTCAGAGGCTGTCCTTTGGGGGACTTGGGCAGAAGGGCAGCTGGAGAAGC
TGGAGATCCACTTTTGTCAGGAAGGTAGTTCCCGTGTTGGCTGTGGCACTTCCACGT
TTGTTTACGGGGGGGGGGGGGTGTTGGGGGGGCAGGGGGGCAGGAGGGATGGAAT
ACCTTGGCTGGGGAGAGGACAGTAGGCCCTGGGGGTGTGGAGCCTGGGCCTGCTCT
ATGGGCGGGGCCTAGAATTCCACAAACTGCTTCTGCCACACTTGCTGGAGGCCCTC
CAGCCCCCATCCCCCAACCCGCCCCCCACACCCTGTCACTTCAGCCTCCCCACTGCT
CCTAGGAGATACCAGCATTGCCAGGACTCTTCTTCCCTGTCTCTCCTCTTCTTCCTCT
GCCCAGGTGTCTCAGGTGGGAGGTGTGTGTGTGGGGAATAGTTAACTCCCTAAGGC
TCTTTTGTAAAGTTTTTGTAGTGATTTTTATGCCACCTGAATAAAGAATGAATGACC
CTGGCTGTTTTGATGTCACTCTGCTGGGCATGGGCTGGAGCAAGGGGGCTGAGGGA
GGCAGGAGGGCCCTGGCGGCACTGAGCCTCACGCTGGGCCCTGACAGCACACAGC
AGGCGGccctctctgccacgtttgtcccctggccacccgccagctcttccccggcgcagtcccctctgaccttgccagccctctctgc
ggccagctccatgccagccgggctccactctctccagaggcctcaagcagccattaacaagggtggctcagaatagcgctggcctccca
gtttggagaaaactggcaattagggagcttgtggagtttctaattacatgtcagtccttctggcaggggcaggcgcccgtcagccagggac
aggctgcagggagcactggttccagctggacgctgtcatgcctgaggatctgggggaaacagttgggcccaccttggcccggggccgt
gggtggtatgcagggccacgtgcgtgggcccttatctgtgggtacagggtgtccctgtggccgtggccacctgaagccccgaagtgcag
tggtcaagagcaagtgcccagggtggactctggcttcctacaaaccagcaatgtggggagacaggtgaaatcgatgatgaggcagagg
gtggttggttagagccgtggtgagaaatagagcagggaaggcctccctgagaaggtggtacgggagcagagacttgaggcaggtgaa
 
ggcaggcatgatataaataaccagggcagagttcctgggtggaagcatgctcagaaggttcaggatggggatccctgggtggcgcagc
ggtttggcgcttgcctttggcccagggcgcgatcctggagacccgggatcaaatcccacgtcgggctcctggtgcatggagcccgcttct
ccctcaacctgtgtctctgcctctctctctctctctgtgactatcataaaaaaaaaaaaaaattagaaggttcaggatgaacagggggtccgt
ggagctagaggggaggggtaggagtgagtcagaggggaacatgtactagtgctttagggccactgtaaagactgttgtttttatcttagga
gtaagaagtaattggatagttaaa (SEQ ID NO:4)
A cDNA sequence of the SERPINF2 gene containing a mutation of interest (c.605 c>t) is shown as SEQ ID NO:5: 
atgggctggctggagcctgagcaaagtgtggggagcaccacagtggggccagagggacattgtgtgcagcagcaaggagcctacag
aggtcaacaacaaggctctccggcccatcccaccaaacctgccgagacctcagggattcacaggcctgtctacccttgtggtcaagacc
agaccaagaaaaggaacatggcgctgctccgggggctcctggtgatcagcttgtcctgcctccaaggctccggcgcggtgttccctcct
gccagcgccatggagcccttggaccagcagctaatgggcgggcagacccaagagaagctgcccccgctcaccctcctcaagttgggc
aaccaggagtccagtggccatattggcctgaagaaggccccaagagactgcaggggagccccaaccccagagcagacccgcagatt
ggcccaggccatgatggccttcaccacagacctgttctccctggtggcccaaaggtccaccagccccaacctaatcctgtcgcctctgag
tgtggccctggcactgtctcacctggcactaggtgctcagaaccagacgctgcggaggttagagcaggtgctgcatgtagactctgggcc
ctgcctcccccacctgctgagccgcctttgccaggacctgggccctggggcattccgattggctgctagaatgtacctgcagaaaggattt
cccatcaaagaggacttcctgaaacaatcagaacagctctttggtgccaagcccatgagcctgaccggaaggaaaggggatgatctgct
aaacatcaaccaatgggtgaaggaggccacagaggggaagattgaggatttcctctcagagctgccagatgacacagtgttgcttctgct
caatgccatccacttccagggtttctggaggagcaagtttgatccaagcctcacccagagagacagtttccacctgaacgagcagttcacg
gtgccagtggacatgatgcacgcccgcacgtaccccctgcgctggttcctgctggagcagcctgagatacaggtggctcatttccccttta
agaacaacatgagctttgtggtcattatgcccacccactttgagtggaacgtgtcccaggtgctggccaacctgagctgggacatcctgca
ccagcccttactgcgagagaaacccaccaaggtccggctgcctaagctgcacctgaactaccagctggacttggtggccaccctcagcc
ggctgggcctgcaggagctgttccaggccccagacctgcgcgggatctccgaccagcgcctggtggtgaccagcgtgcagcaccagt
ctgctctggagctcagtgaggctggtgtggaggcggccgcggccacgggcacggccatgtcccgaatgtctctctcctccttcagcgtca
accgccccttcctcttcttcatcctcgaggacacaacaagcctgcccctgtttgtgggcagtgtgaagaaccccaaccctagtgcgccatg
ggagcgcaaggagcagcaggattcccccgatgacagggactacttccagaaccggaaagcctttccccgcggagacaagcccttcgg
gcctgacttgaaacttgcgcctccctcagaggaggattacccccaacttcatagccccaagtga (SEQ ID NO:5)
An amino acid sequence of the SERPINF2 gene containing a mutation of interest (p.A202V) is shown as SEQ ID NO:6:

MGWLEPEQSVGSTTVGPEGHCVQQQGAYRGQQQGSPAHPTKPAETSGIHRPVYPCGQ
DQTKKRNMALLRGLLVISLSCLQGSGAVFPPASAMEPLDQQLMGGQTQEKLPPLTLLK
LGNQESSGHIGLKKAPRDCRGAPTPEQTRRLAQAMMAFTTDLFSLVAQRSTSPNLILSP
LSVALALSHLALGAQNQTLRRLEQVLHVDSGPCLPHLLSRLCQDLGPGAFRLAARMYL
QKGFPIKEDFLKQSEQLFGAKPMSLTGRKGDDLLNINQWVKEATEGKIEDFLSELPDDT
VLLLLNAIHFQGFWRSKFDPSLTQRDSFHLNEQFTVPVDMMHARTYPLRWFLLEQPEI
QVAHFPFKNNMSFVVIMPTHFEWNVSQVLANLSWDILHQPLLREKPTKVRLPKLHLNY
QLDLVATLSRLGLQELFQAPDLRGISDQRLVVTSVQHQSALELSEAGVEAAAATGTAM
SRMSLSSFSVNRPFLFFILEDTTSLPLFVGSVKNPNPSAPWERKEQQDSPDDRDYFQNRK
AFPRGDKPFGPDLKLAPPSEEDYPQLHSPK (SEQ ID NO:6)

In an embodiment, the animal is a canine. In an embodiment, the canine is selected from the group consisting of a Greyhound, an Irish Wolfhound, a Basenji, an Italian Greyhound, a Galgo Español, an Azawakh, a Scottish Deerhound, a Whippet, a Saluki, a Peruvian Inca Orchid, an English Bulldog, a Shetland Sheepdog, a Jack Russell Terrier, a French Bulldog, a Shiba Inu, a Boston Terrier, a Newfoundland, an Akita, a Golden Retriever, an Alaskan Malamute, an Australian Cattle Dog, a Boxer, a Border Collie, a Yorkshire Terrier, a Cairn Terrier, a Great Pyrenees, a Lhasa Apso, a Miniature Schnauzer, a German Shepherd, an American Staffordshire Terrier, a Pitbull, a Chesapeake Bay Retriever, a Beagle, a Collie, a Labrador Retriever, a Rottweiler, a Chow Chow, and a Mixed Breed.

In an embodiment, the canine is selected from the group consisting of a Scottish deerhound, greyhound, an Irish wolfhound, and a sighthound. In an embodiment, the canine is a Scottish deerhound. In an embodiment, the canine is a greyhound. In an embodiment, the canine is an Irish wolfhound. In an embodiment, the canine is a sighthound.

In an embodiment, the canine is a Basenji. In an embodiment, the canine is an Italian Greyhound. In an embodiment, the canine is a Galgo Español. In an embodiment, the canine is an Azawakh. In an embodiment, the canine is a Whippet. In an embodiment, the canine is a Saluki. In an embodiment, the canine is a Peruvian Inca Orchid. In an embodiment, the canine is an English Bulldog. In an embodiment, the canine is a Shetland Sheepdog. In an embodiment, the canine is a Jack Russell Terrier. In an embodiment, the canine is a French Bulldog. In an embodiment, the canine is a Shiba Inu. In an embodiment, the canine is a Boston Terrier. In an embodiment, the canine is a Newfoundland. In an embodiment, the canine is an Akita. In an embodiment, the canine is a Golden Retriever. In an embodiment, the canine is an Alaskan Malamute. In an embodiment, the canine is an Australian Cattle Dog. In an embodiment, the canine is a Boxer. In an embodiment, the canine is a Border Collie. In an embodiment, the canine is a Yorkshire Terrier. In an embodiment, the canine is a Cairn Terrier. In an embodiment, the canine is a Great Pyrenees. In an embodiment, the canine is a Lhasa Apso. In an embodiment, the canine is a Miniature Schnauzer. In an embodiment, the canine is a German Shepherd. In an embodiment, the canine is an American Staffordshire Terrier. In an embodiment, the canine is a Pitbull. In an embodiment, the canine is a Chesapeake Bay Retriever. In an embodiment, the canine is a Beagle. In an embodiment, the canine is a Collie. In an embodiment, the canine is a Labrador Retriever. In an embodiment, the canine is a Rottweiler. In an embodiment, the canine is a Chow Chow. In an embodiment, the canine is a Mixed Breed.

In an embodiment, the veterinary composition comprises an active ingredient comprising a plasmin inhibitor. As used herein, the term "veterinary acceptable carrier" refers to any agents which do not cause an intolerable side effect and which allow the active ingredients in the veterinary composition to retain their pharmacological activities. A veterinary acceptable carrier includes excipients, emulsifiers, solubilizers, surfactants, buffers, preservatives, and/or other additives which may enhance stability, delivery, absorption, half-life, efficacy, pharmacokinetics, pharmacodynamics, reduce adverse side effect or provide other advantages for veterinary use. In an embodiment, the veterinary acceptable carrier is selected from the group consisting of selected from the group consisting of saline, glucose, alcohols, glycols, esters, amides, and a combination thereof. In an embodiment, the veterinary composition comprises a further active ingredient.

In an embodiment, the veterinary composition is a unit dose. In an embodiment, the veterinary composition is a single unit dose. As used herein, the term "unit dose" is a discrete amount of the veterinary composition comprising a predetermined amount of one or more components. The amount of the components is generally equal to the dosage of the components which would be administered to an animal or a convenient fraction of such a dosage such as, for example, one-half or one-third of such a dosage.

A plasmin inhibitor refers to a composition that provides inhibition of plasmin, in both *in vitro* and *in vivo* settings. For instance, a plasmin inhibitor can bind to the same sites on plasmin to which alpha-2 antiplasmin binds. In an embodiment, the plasmin inhibitor is administered to the animal at a therapeutically effective dose, for example a dose at which plasmin is inhibited.

In an embodiment, the plasmin inhibitor is administered to the animal via an injection. In an embodiment, the injection is an intravenous injection. In an embodiment, the injection is an intradermal injection. In an embodiment, the injection is a subcutaneous injection. In an embodiment, the injection is an intramuscular injection. In an embodiment, the plasmin inhibitor is administered to the animal orally.

In an embodiment, the plasmin inhibitor is administered to the animal every 24 hours. In an embodiment, the plasmin inhibitor is administered to the animal every 12 hours. In an embodiment, the plasmin inhibitor is administered to the animal every 8 hours. In an embodiment, the plasmin inhibitor is administered to the animal every 6 hours. In an embodiment, the plasmin inhibitor is administered to the animal every 6 to 8 hours. In an embodiment, the plasmin inhibitor is administered to the animal every 8 to 12 hours.

In an embodiment, the plasmin inhibitor is selected from the group consisting of an aminocaproic acid, a tranexamic acid, a yunnan baiyao, and any combination thereof. In an embodiment, the plasmin inhibitor is an aminocaproic acid. In an embodiment, the aminocaproic acid is administered at a dose of 25 mg/kg to 50 mg/kg. In an embodiment, the aminocaproic acid is administered at a dose of 25-50 mg/kg every 6 to 8 hours. Aminocaproic acid is also known as ε-aminocaproic acid, ε-Ahx, and 6-aminohexanoic acid and has a structure of:

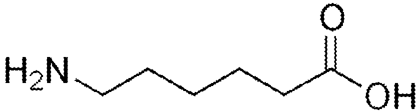

In an embodiment, the plasmin inhibitor is a tranexamic acid. In an embodiment, the tranexamic acid is administered at a dose of 10 mg/kg to 15 mg/kg. In an embodiment, the tranexamic acid is administered at a dose of 10 mg/kg to 15 mg/kg every 8 to 12 hours. Tranexamic acid is also known as TXA and has a structure of:

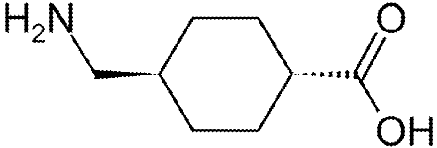

In an embodiment, the plasmin inhibitor is a yunnan baiyao. In an embodiment, the yunnan baiyao is administered at a dose of 12.5 mg/kg to 25 mg/kg. In an embodiment, the yunnan baiyao is administered at a dose of 12.5 mg/kg to 25 mg/kg every 12 hours. Yunnan baiyao is a traditional Chinese herbal remedy and can comprise several compounds.

In an embodiment, the animal is administered a second therapeutic agent.

In a further illustrative aspect, a method for detecting sensitivity to bleeding in an animal is provided. The method comprises the step of determining if a genetic mutation in a SERPINF2 gene is present in the animal, wherein the presence of the genetic mutation in the SERPINF2 gene indicates that the animal is sensitive to bleeding. The previously described embodiments of the method of treating bleeding in an animal are applicable to the method for detecting sensitivity to bleeding in an animal described herein.

In an embodiment, the method further comprises the step of making a breeding decision based on the presence of the genetic mutation in the SERPINF2 gene. For instance, if a genetic mutation in the SERPINF2 gene is detected, an affirmative breeding decision based on the presence of the genetic mutation could be made. Alternatively, if a genetic mutation in the SERPINF2 gene is not detected, an affirmative breeding decision based on the presence of the genetic mutation could be made.

In a further illustrative aspect, method for making a breeding decision for an animal is provided. The method comprises the steps of determining if a genetic mutation in a SERPINF2 gene is present in the animal, wherein the presence of the genetic mutation in the SERPINF2 gene indicates that the animal is sensitive to bleeding, and making the breeding decision based on the presence of the genetic mutation in the SERPINF2 gene. The previously described embodiments of the method of treating bleeding in an animal are applicable to the method for making a breeding decision for an animal described herein.

### EXAMPLE 1

### Methods for Identification of Genetic Mutations in Canines

For the instant example, Scottish deerhounds with delayed postoperative bleeding and matched controls were identified through a survey distributed to members of the Scottish Deerhound Club of America. A subsection of this survey requested information regarding the surgical history of the dogs, occurrence of postoperative bleeding complications, and perioperative administration of antifibrinolytic drugs (EACA or tranexamic acid). A diagnosis of delayed postoperative bleeding, defined as unexpected bleeding from a surgical wound starting 1 to 4 days after the procedure, was based on review of the owner's description of the event, the veterinary medical record (if available), and therapeutic response to administration of antifibrinolytic drugs (if used). Dogs were excluded if the bleeding event occurred on the same day of surgery, or after more than 4 days following surgery.

Control dogs were included if they had undergone a surgical procedure where the owners reported no evidence for delayed postoperative bleeding. They were excluded as controls if antifibrinolytic drugs had been administered prophylactically. DNA samples from cases and controls were obtained either directly by cheek swab collection from the dogs by the owners or were archived samples obtained from the Canine Health Information Center (CHIC) DNA repository, or PennGen Laboratories (University of Pennsylvania).

### Gene-capture sequencing

Seven candidate genes involved in regulating fibrinolysis and clot strength (see Figure 4) were sequenced in DNA samples from 4 affected and 6 control dogs by a genomics service using a proprietary gene-capture technology (Rapid Genomics LLC, Gainesville, FL). The genes (and their protein products) included *SERPINF2* (alpha-2 antiplasmin), *SERPINE1* (plasminogen activator inhibitor 1), *SERPINB2* (plasminogen activator inhibitor 2), *A2M* (alpha-2 macroglobulin), *CPB2* (carboxypeptidase B2), *F13A1* (coagulation factor XIIIA) and *F13B* (coagulation factor XIIIB).

Gene regions targeted for sequencing included all exons as well as the 5'- and 3'-untranslated regions, based on NCBI Annotation Release 105 of the available public canine genome assembly. For the 10 DNA samples that were sequenced, the median probe depth for individual samples ranged from 25 to 521, with a median value of 53 among all samples. Reads were mapped to the canine reference genome (CanFam3.1) using Bowtie for Illumina (Galaxy Version 1.1.2). Mapped reads were then visualized with Golden Helix GenomeBrowse version 2.1.2 using the NCBI Refseq 104 Genes annotation and the NCBI dbSNP version 146 variant annotation. Sequence variants in the coding region were identified by comparing the sequences of mapped reads for each DNA sample with the annotated gene reference sequence. PolyPhen2 was used to evaluate the effect of identified missense SNVs on protein function.

### SERPINF2 c.605 C>T and F7 c.407 G>A genotyping

A custom allele discrimination assay (Applied Biosystems TaqMan SNP Genotyping Assay, Thermo Fisher Scientific, Waltham MA) was developed to genotype DNA samples for the *SERPINF2* c.605 C>T mutation. The primers and probes were 5'-ACG CTG CGG AGG TTA GAG-3' (forward primer; SEQ ID NO:7), 5'-CCC AGG TCC TGG CAA AGG-3' (reverse primer; SEQ ID NO:8), 5'-CCA GAG TCT GCA TGC AG-3' (C-allele probe labelled with VIC dye; SEQ ID NO:9), and 5'-CCA GAG TCT ACA TGC AG-3' (T-allele probe labelled with FAM dye; SEQ ID NO:10).

Assays were performed according to the manufacturer's directions using a real-time PCR instrument (CFX96 Touch, Bio-Rad, Hercules, CA). Assay accuracy was verified by genotyping the same case/control Scottish Deerhound DNA samples that had been sequenced using an independent gene capture approach (see above). F7 c.407 G>A mutation genotype was determined by Sanger sequencing of PCR product generated using primers 5'-ATC AAA CCT CAG CGG GGC TGG-3' (Pri-1291; SEQ ID NO: 11) and 5'-GGG CTT GTT TCC GAG CGG G-3' (Pri-1292; SEQ ID NO: 12).

### Collection of plasma samples for plasmin inhibition assay

Healthy NGA-registered greyhounds (N = 19) aged between 1 and 10 years old were recruited from eastern Washington state and northern Idaho. Health was ascertained by review of the dog's medical history and a physical exam. Studied dogs included 6 neutered males, one intact male, and 12 spayed females aged between 2 and 9 years (average 4.9 years). Blood samples (3 mL) were collected by a single venipuncture from the cephalic or saphenous veins, immediately transferred to tubes containing sodium citrate anticoagulant and centrifuged. The plasma layer was removed and stored at -80°C until analysis. Cheek swab DNA samples were also collected from each dog and used to determine *SERPINF2* c.605 C>T genotype.

### Plasmin inhibition assay

A microplate-based colorimetric kinetic assay was developed and validated to measure the potency of inhibition of canine plasmin by plasma based on a previously published method. Briefly, 2 µL of canine plasma diluted to 50 µL in phosphate-buffered saline (PBS) was added on ice to 96-well UV clear nonbinding plates (Cat.#655901, Greiner Bio-One, Monroe, NC). Then, 50 µL of canine plasmin (Cat.# DPLM, Molecular Innovation, Novi, MI) at 100 nM concentration in 50 µL PBS was added, mixed by pipetting, and incubated at 37°C for 5 min. To this, 100 µL of prewarmed plasmin substrate (D-VLK-pNA, Molecular Innovation, Novi, MI) at 200 µM concentration in PBS was added, mixed, and immediately placed in a colorimetric plate reader (SpectraMax i3, Molecular Devices, San Jose, CA) set at 37°C. Absorbance at 405 nM was then monitored in each well over the next 5 minutes. Plasmin hydrolysis activity for each well was calculated from the fitted linear slope of the absorbance versus time curve over 5 minutes.

Preliminary studies confirmed concentration-dependent inhibition of plasmin activity by the antifibrinolytic aprotinin (Cat.# A1153, Sigma Aldrich, St Louis, MO) with an IC50 of approximately 500 nM (see Figure 1). Plasma collected in sodium citrate and pooled from 2 male and 2 female beagle dogs was obtained from a commercial vendor (Biochemed Services, Winchester, VA). For quantitation purposes, this pooled canine plasma was used as a calibration standard to determine relative plasmin inhibition potency (as a percentage of the standard) for plasma samples collected from *SERPINF2* genotyped greyhound dogs. Calibration curves were prepared using 0, 0.5, 1, 1.5, 2, 3, and 4 µL of pooled plasma standard. Plasmin activity for each sample with added pooled plasma standard was calculated as the percentage of plasmin activity without added plasma (control).

Plots of plasma standard volume versus log-transformed percent plasmin activity were consistently linear (R² >0.99) over the assay range (0 µL to 4 µL) (see Figure 2). Preliminary studies showed that 2 µL of pooled plasma standard consistently showed about 50% inhibition of plasmin activity compare to samples without added plasma. Consequently, the inhibition observed by 2 µL of pooled plasma standard was arbitrarily set to a value of 100%. The inhibition potency of unknown plasma samples (2 µL added per well) was calculated from the slope and intercept of the standard curve and expressed as a percentage of the pooled plasma standard. The final value for each sample represented the average of 5 independent experiments conducted in triplicate on separate days. Within assay variation (expressed as a coefficient of variation; CV%) averaged 8%, while between assay variation averaged 13% for all samples analyzed.

### Dog breed representative DNA samples

Stored DNA samples (N = 2,298) from client-owned dogs were retrieved from the Washington State University Veterinary Teaching Hospital Patient DNA Bank and the Comparative Pharmacogenomics Laboratory Sighthound DNA Bank. DNA was extracted from buccal swab samples obtained by the hospital staff or by the dog's owner. Hospital patient samples were from dogs living in the Pacific Northwest of the United States, while the Sighthound DNA bank samples were obtained primarily by mail from dogs living throughout the United States. For hospital bank samples, the dog's breed was based on owner designation, whereas Sighthound Bank samples were verified by breed registration information. The 2,298 DNA samples represented 75 different dog breeds with a minimum of 10 DNA samples per breed and included 156 samples from mixed-breed dogs. The designation of a breed as belonging to the 'Sighthound' group was based on breed inclusion criteria for Sighthounds established by the American Kennel Club. Samples from greyhound dogs were divided into two breed subgroups based on whether they were identified by their owners as dogs bred for racing and registered with the National Greyhound Association (NGA; N = 197) or were dogs bred for other purposes and registered with the American Kennel Club (AKC; N = 64).

### EXAMPLE 2

### Identification of Delayed Postoperative Bleeding Cases and Controls

The instant example utilizes the methods described in Example 1 to identify delayed postoperative bleeding cases in canines and controls. Completed health surveys from the owners of 260 Scottish deerhounds were reviewed to identify delayed postoperative bleeding cases and controls. A flowchart illustrating the process used to select cases and controls is shown in Figure 3. Of the original 260 dogs evaluated, 105 dogs (40%) were identified that had undergone at least one surgical procedure, while 21 dogs (8%) had received two or more surgeries. Nine of 105 dogs (9%) with a surgical history were reported to have experienced postoperative bleeding and/or excessive bruising at least once after a surgical procedure. Of these, bleeding into the abdomen was first diagnosed within 8 hours after surgery in two dogs, while bleeding and/or bruising was not evident until at least the morning after surgery in the remaining 7 dogs. These latter 7 dogs were considered delayed postoperative bleeding cases and all had DNA samples available for case-control analysis. Of the 96 dogs that had undergone surgery without evidence for postoperative bleeding, 25 dogs (26%) had received prophylactic treatment with EACA, while 71 dogs (74%) had not received any antifibrinolytic drug. These latter 71 dogs were considered delayed postoperative bleeding controls. Of the 71 control dogs, 55 dogs had DNA samples available for case-control genetic analysis.

Case information for the seven dogs (SDH-1 to SDH-7) that met the inclusion/exclusion criteria for delayed postoperative bleeding are shown in Table 1.

**Table 1. Scottish deerhound cases with delayed postoperative bleeding (F=female, M=male)**

| **Dog ID** | **Sex** | **Surgical procedure/s** | **Prophylactic antifibrinolytic?** | **Delayed bleeding?** | **Therapeutic antifibrinolytic?** | **Outcome** |
|---|---|---|---|---|---|---|
| **SDH-1** | F | Spay for pyometra | No | Yes | No | Survived after 3 days of supportive care |
| **SDH-2** | M | Splenectomy for splenic torsion and prophylactic gastropexy | No | Yes | Yes | Responded to antifibrinolytic and recovered |
| **SDH-3** | M | Cryptorchid castration | No | Yes | Yes | Responded to antifibrinolytic and recovered |
| | | Splenectomy for splenic hematoma | Yes | No | No | No complications |
| **SDH-4** | F | Spay | No | No | No | No complications |
| | | Left front leg fracture repair | No | No | No | No complications |
| | | Left front leg amputation | No | Yes | Yes | Responded to antifibrinolytic and recovered |
| **SDH-5** | F | Laparoscopic oophorectomy | No | Yes | Yes | Responded to antifibrinolytic and recovered |
| **SDH-6** | F | Drain abscess and remove foreign body | Yes | No | No | No complications |
| | | Remove sebaceous cyst | No | Yes | Yes | Responded to antifibrinolytic and recovered |
| **SDH-7** | F | Spay | No | Yes | No | Died 48 h after routine spay despite supportive care. |

Surgical procedures associated with delayed bleeding ranged from quite invasive procedures with the potential to cause extensive intraoperative bleeding, including splenectomy (SDH-2) and limb amputation (SDH-4), to moderately invasive procedures, including spays (SDH-1 and SDH-7), oophorectomy (SDH-5), and castration (SDH-3). One dog had experienced delayed bleeding after a relatively minor procedure involving removal of a sebaceous cyst from the skin (SDH-6). In addition to supportive care (primarily administration of intravenous fluids), most dogs (SDH-1 to SDH-6) with delayed postoperative bleeding were also treated with and responded positively to an antifibrinolytic drug. Specifically, bleeding was reported to have stopped within hours of drug administration. EACA (oral or injectable) was used in all instances. However, two dogs were not treated with any antifibrinolytic drug (SDH-1 and SDH-7). One of these dogs (SDH-1) recovered after 3 days of supportive care, while the other dog (SDH-7) died 2 days after a routine spay despite similar supportive care. Although a necropsy examination was performed on this latter dog (SDH-7), the report could not be obtained by the owner for review.

Complete and detailed veterinary medical records were available to review for two dogs (SDH-2 and SDH-3). SDH-2 was a 5-year-old male Scottish deerhound who underwent an exploratory laparotomy for symptoms including abdominal pain and radiographic evidence of splenic enlargement. Splenic torsion was diagnosed during surgery. A splenectomy was performed, as well as a prophylactic gastropexy. Recovery was uneventful until the morning after the procedure when both the hematocrit and the platelet count had significantly decreased (relative to immediate postoperative values) and continued to decrease throughout the day (see Table 2).

**Table 2. Hematologic values in a Scottish deerhound dog (SDH-2) with delayed postoperative bleeding after an emergency splenectomy and prophylactic gastropexy. The bottom row displays reference intervals established for healthy North American Scottish deerhounds.**

| **Sample collection time** | **Hematocrit (%)** | **Platelet count (x 10⁹/L)** |
|---|---|---|
| **Day before surgery** | 54.7 | 115 |
| **Day of surgery** - **postoperative** | 48.2 | 95 |
| **Day 1 - morning (#1)** | 39.5 | 66 |
| **Day 1 - morning (#2)** | 31.9 | 73 |
| **Day 1 - evening (after aminocaproic acid)** | 34.4 | 31 |
| **Day 2** | 41.7 | 125 |
| **Day 5** | 51.9 | 371 |
| **Week 5** | 50.4 | 276 |
| **Breed-specific reference interval** [8] | 44 - 62 | 37 - 270 |

Frank blood was noted to be oozing from the abdominal incision. A moderate amount of fluid was also found in the abdominal cavity by ultrasound examination. EACA solution (750 mg) was diluted in Ringer's lactate solution and administered as an intravenous infusion. Both hematocrit and platelet counts significantly increased by the next morning (Day 2) and were within their normal range by Day 5. The dog recovered completely from the surgical procedure and was in good health at 8 years of age.

SDH-3 was a 1-year-old male Scottish deerhound diagnosed with unilateral cryptorchidism. Surgical castration was performed. Recovery was uncomplicated and the dog returned home the next day. However late on the second day after the procedure very heavy bleeding from the surgical site was observed by the owner. The dog was returned to the clinic where an exploratory surgery of the wound was performed. No single source of bleeding was identified, and the area was extensively cauterized. Following this surgery, in addition to intravenous fluids, antibiotics, and a single vitamin K injection, the dog was administered 500 mg of EACA orally, which was continued every 8 hours for 5 days. No further bleeding was observed by the owner and the dog recovered completely. Four years later, the same dog underwent an elective splenectomy surgery for suspected hemangiosarcoma. The dog received 500 mg of EACA as an intravenous infusion during the procedure, which was followed by a 500 mg oral dose every 8 hours for 5 days. No excessive bruising or delayed postoperative bleeding were observed and the dog completely recovered from the procedure. Examination of the spleen revealed multiple benign splenic hematomas rather than hemangiosarcoma. The dog was in good health at 8 years of age.

Two other dogs (SDH-4 and SDH-6) also had multiple surgical procedures with differing outcomes. SDH-4 was spayed at 1 year of age and had a surgical stabilization of a left front leg fracture at 6 years of age without evidence for delayed postoperative bleeding. However, at 6.5 years of age the dog's left foreleg was amputated after a diagnosis of osteosarcoma. The dog underwent the procedure without complication and was discharged the second day after surgery. Nevertheless, within 2 hours of returning home the owner noticed the appearance of severe bruising of the chest and abdomen with edema. The dog was readmitted where a low hematocrit value (26% compared to 61% before surgery) was found. The hematocrit continued to fall to 15% overnight. Based on a diagnosis of delayed postoperative bleeding, the dog was treated with a continuous intravenous infusion of EACA, as well as several transfusions of whole blood and plasma. The dog was discharged after 5 days with a hematocrit of 36%, which increased to 60% after one month. The dog had no further complications from the procedure. However, it was euthanized 6 months later because of paralysis associated with tumor metastasis to the spinal cord.

SDH-6 was surgically treated for a skin abscess resulting from a foreign body when 5 years old. The dog had received a pre-operative oral dose of EACA. No postoperative bleeding or bruising was observed. However, when 7 years old, a cutaneous sebaceous cyst was removed surgically without administration of prophylactic EACA. The next day after discharge, the owner observed excessive postoperative bleeding from the incision site. The dog was subsequently treated with orally administered EACA and the bleeding resolved. The dog recovered without further complication and was in good health at 9 years of age.

A further 55 Scottish deerhounds (SDH-C-1 to SDH-C-55) with available DNA samples were identified as controls for case-control genetic association analysis. The majority of dogs had undergone surgical desexing procedures (15 castrations, 13 spays, and 3 ovariectomies) or Caesarean section (n = 15). Seven dogs were treated for major skin lacerations. The remaining 15 dogs underwent major abdominal or orthopaedic procedures, including cystotomies (n = 4), gastric dilatation/volvulus surgery (n = 2), splenectomy (n = 1), fracture repair (n = 4), anterior cruciate repair (n=1), patellar luxation correction (n=1), Achilles tendon repair (n = 1), and a digit amputation (n = 1). Thirteen dogs had multiple surgical procedures performed.

Gene-capture sequencing was used in an exploratory analysis to discover coding region variants in 7 candidate genes encoding proteins known to negatively regulate the fibrinolysis pathway to evaluate if mutations in one or more of these genes led to enhanced fibrinolysis (see Figure 4). This discovery analysis used a subset of case (n = 4; SDH-1 to SDH-4) and control (n = 6; SDH-C-1 to SDH-C-6) DNA samples, representing the first 4 cases and the first 6 controls with available DNA samples. Genes analyzed included *SERPINF2, A2M, SERPINE1, SERPINB2,* and *CPB2,* which encode for fibrinolysis inhibitors; as well as *F13A1* and *F13B,* which encode for clot stabilizers (see Figure 4).

Ten single nucleotide variants (SNVs) were identified in 6 of the genes, while *F13B* contained no coding region variants (see Figure 5). All SNVs except for *A2M* c.2194 G>A were found in the dbSNP v.146 database. Four SNVs were nonsynonymous, including one in *SERPINF2* (c.605 C>T, p.A202V) and all three found in *A2M* (c.2096 C>T, p.A699V; c.2194 G>A, p.E732K; c.3586 C>G, p.R1196G). The results of PolyPhen-2 computational analysis of the effect of each amino acid substitution on protein function (see Figure 5). PolyPhen-2 scores can range from 0.0 to 1.0 with higher scores considered more disruptive to protein function. Interestingly, the *SERPINF2* p.A202V substitution showed the highest PolyPhen-2 score (0.31; sensitivity 0.9, specificity 0.89) out of the 4 amino acid changes analyzed.

The genotypes for each of the SNVs in the discovery subset of cases and controls are shown in Figure 6. Comparison of the proportion of dogs with at least one variant allele between case and control groups showed a difference for both of the *SERPINF2* SNVs (P = 0.012; Z-test), but not for any other SNV evaluated (P > 0.05). Specifically, all cases were either heterozygous or homozygous variant genotype for *SERPINF2* c.605 C>T and c.696 G>A, while all control dogs were homozygous reference genotype for both mutations.

*SERPINF2* c.605 C>T was chosen for further study by case-control genotype association analysis since this variant showed the greatest effect on protein function and the strongest association with delayed bleeding phenotype in the discovery sample DNA subset.

### EXAMPLE 3

### Association of Genotype with Delayed Postoperative Bleeding

The instant example utilizes the methods described in Examples 1 and 2 to demonstrate that *SERPINF2* c.605 C>T genotype is associated with delayed postoperative bleeding. Briefly, the case-control genotype-phenotype association analysis was used to evaluate whether *SERPINF2* c.605 C>T genotype was correlated with delayed postoperative bleeding phenotype. *SERPINF2* c.605 C>T genotype results obtained by gene-capture sequencing were confirmed using a Taqman allele discrimination assay and extended to include all cases (n = 7) and controls (n = 55). As shown in Table 3, the variant *SERPINF2* c.605 T allele frequency in cases was over 5 times that of control dogs (allelic OR = 16; 95% CI = 4.4 - 57; P < 0.0001).

**Table 3. SERPINF2 c.605 C>T genotype results for 62 Scottish deerhounds with known postoperative bleeding phenotype (7 cases and 55 controls).**

| **Phenotype** | ***SERPINF2* c.605 C>T genotype (N dogs)** | | | **Variant allele frequency (%)** | **Allelic OR (95% CI)** | **P-value** |
|---|---|---|---|---|---|---|
| | **C/C** | **C/T** | **T/T** | | | |
| **Case** | 0 | 4 | 3 | 71 | 16 (4.4 - 57) | <0.0001 |
| **Control** | 40 | 15 | 0 | 14 | Reference | |

None of the cases had the reference (*SERPINF2* c.605 C/C) genotype, while none of the control dogs had the homozygous variant (*SERPINF2* c.605 T/T) genotype. Compared to dogs with the reference *SERPINF2* c.605 C/C genotype, the odds for postoperative bleeding were significantly higher for dogs with the heterozygous C/T genotype (genotypic OR = 24; 95% CI = 1.2 - 463; P = 0.034) and with the homozygous variant T/T genotype (genotypic OR = 567; 95% CI = 9.7 - 33177; P = 0.0023).

*F7* c.407 G>A genotypes were also determined for the same case and control dogs (Table 4). In contrast to the *SERPINF2* genotype results, there was no difference in the frequency of the variant F7 c.407 A allele in cases versus control dogs (allelic OR = 0.98; 95% CI = 0.2 - 4.8; P = 0.98).

**Table 4. F7 c.407 G>A genotype results for 61 Scottish deerhounds with known postoperative bleeding phenotype (7 cases and 55 controls).**

| **Phenotype** | ***F7* c.407 G>A genotype (N dogs)** | | | **Variant allele frequency (%)** | **Allelic OR (95 % CI)** | **P-value** |
|---|---|---|---|---|---|---|
| | **G/G** | **G/A** | **A/A** | | | |
| **Case** | 5 | 2 | 0 | 14 | 0.98 (0.2 - 4.8) | 0.98 |
| **Control** | 43 | 8 | 4 | 15 | Reference | |

### EXAMPLE 4

### Association of SERPINF2 c.605 TT Genotype with Lower Plasma Antiplasmin Activities

The instant example utilizes the methods described in Examples 1 and 2 to demonstrate that *SERPINF2* c.605 TT genotype is associated with lower plasma antiplasmin activities. Given the association of the *SERPINF2* c.605 T allele with delayed postoperative bleeding, the function of alpha-2 antiplasmin (encoded by *SERPINF2*) was investigated to see if it would be decreased in the plasma of dogs with this variant. Since all commercial antiplasmin assays currently use human plasmin, a plasmin inhibition assay was developed using purified canine plasmin and validated with pooled Beagle dog plasma as a reference standard.

Retired racing greyhounds (greyhounds registered with the National Greyhound Association [NGA]) were studied because they have a relatively high *SERPINF2* c.605 T allele prevalence and are reportedly predisposed to delayed postoperative bleeding. Out of 19 greyhounds genotyped, 3 dogs were *SERPINF2* c.605 C/C, 10 dogs were *SERPINF2* c.605 C/T and 6 dogs were *SERPINF2* c.605 T/T. As shown in Figure 7, there were significant differences in plasma antiplasmin activities between genotype groups (P = 0.046; ANOVA). Pairwise comparisons showed that mean (± SD) antiplasmin activity was significantly lower in c.605 T/T plasma (100 + 10%) compared with c.605 C/T plasma (113 ± 8%) (P = 0.044; Holm-Sidak test). Average activity of c.605 T/T plasma was also lower than c.605 C/C plasma (107 + 9%), but did not achieve statistical significance (P > 0.05). Given the relatively small number of c.605 C/C samples, the results from c.605 C/C and C/T samples were combined and compared with the c.605 T/T samples. Again, activities were significantly lower for c.605 T/T samples (100 + 10%) compared with the c.605 C/T or C/C plasma samples (111 ± 8%) (P = 0.021; Student's t-test).

The instant example demonstrates that reduced plasma antiplasmin activities in greyhounds with the *SERPINF2* c.605 T/T genotype. This finding suggests that this variant (or perhaps another variant in close genetic linkage) negatively affects expression or function of the antiplasmin protein.

### EXAMPLE 5

### Identification of SERPINF2 c.605 T Allele in Dog Breeds Susceptible to Delayed Postoperative Bleeding

The instant example utilizes the methods described in Example 1 to demonstrate that the *SERPINF2* c.605 T allele is primarily found in breeds susceptible to delayed postoperative bleeding. If the *SERPINF2* c.605 C>T variant was causally linked to delayed postoperative bleeding, it would follow that the prevalence of this variant should be highest in those breeds known to be susceptible to this disorder, including retired racing greyhounds and Scottish deerhounds, as well as other sighthound dog breeds. Consequently, the prevalence and breed distribution of the variant *SERPINF2* c.605 T allele was determined in 2,298 dogs representing 75 dog breeds (including 20 sighthound breeds) and 156 mixed-breed dogs. The *SERPINF2* c.605 T allele frequencies are shown in Figures 8A-8B.

The *SERPINF2* c.605 T allele was found in a higher proportion of sighthound dog breeds (15 of 20; 75%; see FIG. 8A) compared with non-sighthound breeds (28 of 55; 51%; see FIG. 8B). Notably, the mean (± SE) allele frequency in sighthound dog breeds (21 ± 5%) was over 4-fold higher (P < 0.001, Mann-Whitney U test) compared with non-sighthound breeds (4.0 ± 0.9%). Within individual sighthound dog breeds, Scottish deerhound showed a moderate variant allele prevalence (25%) with 7 sighthound breeds showing higher frequencies and 12 sighthound breeds showing lower frequencies. American Kennel Club (AKC) registered and NGA registered greyhounds showed the highest (80%) and fourth highest (43%) variant allele frequencies, respectively. Other breeds with relatively high prevalence (>40%) included Irish wolfhound (50%), basenji (46%), and Italian greyhound (43%). Among non-sighthound dog breeds, only English bulldog (30%) and Shetland sheepdog (24%) showed allele frequencies that were similar to or higher than Scottish deerhound. All remaining non-sighthound breeds showed substantially lower allele prevalence (15% or less).

### EXAMPLE 6

### Genetic Sample Acquisition and Testing

The instant example utilizes genetic samples (e.g., DNA samples) obtained from animals for utilization according to the present disclosure. For instance, genetic samples can be collected from an animal via a swab or via blood according to general procedures known in the art. Thereafter, the genetic samples can be tested in a laboratory or other clinic.

For example, DNA can be extracted from the genetic sample and then tested via a PCR procedure. For instance, a Taqman SNP allele discrimination assay can be performed via PCR, including by real-time PCR. For this example, positive controls can be utilized comprising DNA samples from dogs with a known genotype (confirmed by Sanger sequencing). Further, negative controls can be utilized comprising samples with no DNA.

The following primers and probes can be utilized according to the instant example:
Forward Primer Sequence: ACGCTGCGGAGGTTAGAG (SEQ ID NO: 13)
Reverse Primer Sequence: CCCAGGTCCTGGCAAAGG (SEQ ID NO:14)
Reporter 1 Sequence: CCAGAGTCTGCATGCAG (SEQ ID NO:15)
Reporter 2 Sequence: CCAGAGTCTACATGCAG (SEQ ID NO:16)

Following testing, genetic results of the animals can be reported as i) Normal/Normal (WT), ii) Normal/Mutant (HET), or iii) Mutant/Mutant (VAR).

Without being bound to any theory, it is believed that in Scottish deerhounds a dog identified as "VAR" has over a 500 times higher risk (compared with "WT" dogs) for developing delayed post-operative bleeding following surgery. Accordingly, it is believed that a prophylactic treatment with a plasmin inhibitor is beneficial to prevent post-operative bleeding in these dogs.

Without being bound to any theory, it is believed that in Scottish deerhounds a dog identified as "HET" dogs has a 25 times higher risk (compared with "WT" dogs) for developing delayed post-operative bleeding following surgery, especially if the dog is older in age. Consequently, it is believed that a prophylactic treatment with a plasmin inhibitor is beneficial to prevent post-operative bleeding in these dogs.

## Claims

1. A method for detecting sensitivity to bleeding in a canine animal, said method comprising the step of:
- determining if a genetic mutation in a SERPINF2 gene is present in the animal, wherein the presence of the genetic mutation in the SERPINF2 gene indicates that the animal is sensitive to bleeding, wherein the genetic mutation in the SERPINF2 gene is c.605 C>T or p.A202V.

2. The method of claim 1, wherein the method further comprises the step of making a breeding decision based on the presence of the genetic mutation in the SERPINF2 gene.

3. A method for making a breeding decision for a canine animal, said method comprising the steps of:
- determining if a genetic mutation in a SERPINF2 gene is present in the animal, wherein the presence of the genetic mutation in the SERPINF2 gene indicates that the animal is sensitive to bleeding, wherein the genetic mutation in the SERPINF2 gene is c.605 C>T or p.A202V, and
- making the breeding decision based on the presence of the genetic mutation in the SERPINF2 gene.

4. A veterinary composition for use in treating bleeding in a canine animal wherein it has been determined that a genetic mutation in a SERPINF2 gene is present in the animal, wherein the genetic mutation in the SERPINF2 gene is c.605 C>T or p.A202V.

5. The method of claim 1, wherein the bleeding in the animal is delayed bleeding.

6. The method of claim 1 or 3 or the veterinary composition for the use of claim 4 wherein the canine is:
(a) selected from the group consisting of a Greyhound, an Irish Wolfhound, a Basenji, an Italian Greyhound, a Galgo Español, an Azawakh, a Scottish Deerhound, a Whippet, a Saluki, a Peruvian Inca Orchid, an English Bulldog, a Shetland Sheepdog, a Jack Russell Terrier, a French Bulldog, a Shiba Inu, a Boston Terrier, a Newfoundland, an Akita, a Golden Retriever, an Alaskan Malamute, an Australian Cattle Dog, a Boxer, a Border Collie, a Yorkshire Terrier, a Cairn Terrier, a Great Pyrenees, a Lhasa Apso, a Miniature Schnauzer, a German Shepherd, an American Staffordshire Terrier, a Pitbull, a Chesapeake Bay Retriever, a Beagle, a Collie, a Labrador Retriever, a Rottweiler, a Chow Chow, and a Mixed Breed;
(b) selected from the group consisting of a Scottish deerhound, greyhound, an Irish wolfhound, and a sighthound;
(c) a Scottish deerhound;
(d) a greyhound;
(e) an Irish wolfhound; or
(f) a sighthound.

7. The veterinary composition for the use of claim 4 wherein the animal is treated with the veterinary composition:
(a) as a prophylactic treatment;
(b) before a trauma inducing incident of the animal; or
(c) after a trauma inducing incident of the animal.

8. The veterinary composition for the use of claim 4 wherein the bleeding in the animal is:
(a) delayed bleeding;
(b) delayed bleeding associated with a surgery performed on the animal; or
(c) subsequent to a surgical procedure.

9. The veterinary composition for the use of claim 4 wherein the genetic mutation in the SERPINF2 gene indicates that the animal is sensitive to experience delayed bleeding.

10. The veterinary composition for the use of claim 4 wherein the veterinary composition comprises an active ingredient comprising a plasmin inhibitor.

11. The veterinary composition for the use of claim 10 wherein the plasmin inhibitor is:
(a) administered to the animal at a therapeutically effective dose;
(b) administered to the animal via an injection; or
(c) administered to the animal orally.

12. The veterinary composition for the use of claim 10 wherein the plasmin inhibitor is:
(a) selected from the group consisting of an aminocaproic acid, a tranexamic acid, a yunnan baiyao, and any combination thereof;
(b) an aminocaproic acid;
(c) a tranexamic acid; or
(d) a yunnan baiyao.

13. The veterinary composition for the use of claim 12 wherein the plasmin inhibitor is:
(a) an aminocaproic acid wherein the aminocaproic acid is administered at dose of 25 mg/kg to 50 mg/kg or at a dose of 25-50 mg/kg every 6-8 hours;
(b) a tranexamic acid wherein the tranexamic acid is administered at dose of 10 mg/kg to 15 mg/kg or at a dose of 10 mg/kg to 15 mg/kg every 8-12 hours;
(c) a yunnan baiyao wherein the yunnan baiyao is administered at dose of 12.5 mg/kg to 25 mg/kg or at a dose of 12.5 mg/kg to 25 mg/kg every 12 hours.

## Patentansprüche

1. Verfahren zum Detektieren einer Anfälligkeit für Blutungen bei einem Hundetier, wobei das Verfahren den folgenden Schritt umfasst:
- Bestimmen, ob eine genetische Mutation in einem SERPINF2-Gen bei dem Tier vorliegt, wobei das Vorliegen der genetischen Mutation in dem SERPINF2-Gen darauf hinweist, dass das Tier anfällig für Blutungen ist, wobei die genetische Mutation in dem SERPINF2-Gen c.605 C>T oder p.A202V ist.

2. Verfahren nach Anspruch 1, wobei das Verfahren ferner den Schritt eines Treffens einer Zuchtentscheidung basierend auf dem Vorliegen der genetischen Mutation in dem SERPINF2-Gen umfasst.

3. Verfahren zum Treffen einer Zuchtentscheidung für ein Hundetier, wobei das Verfahren die folgenden Schritte umfasst:
- Bestimmen, ob eine genetische Mutation in einem SERPINF2-Gen bei dem Tier vorliegt, wobei das Vorliegen der genetischen Mutation in dem SERPINF2-Gen darauf hinweist, dass das Tier anfällig für Blutungen ist, wobei die genetische Mutation in dem SERPINF2-Gen c.605 C>T oder p.A202V ist, und
- Treffen der Zuchtentscheidung basierend auf dem Vorliegen der genetischen Mutation in dem SERPINF2-Gen.

4. Veterinärmedizinische Zusammensetzung zur Verwendung beim Behandeln von Blutungen bei einem Hundetier, wobei bestimmt wurde, dass eine genetische Mutation in einem SERPINF2-Gen bei dem Tier vorliegt, wobei die genetische Mutation in dem SERPINF2-Gen c.605 C>T oder p.A202V ist.

5. Verfahren nach Anspruch 1, wobei es sich bei den Blutungen bei dem Tier um Spätblutungen handelt.

6. Verfahren nach Anspruch 1 oder 3 oder veterinärmedizinische Zusammensetzung zur Verwendung nach Anspruch 4, wobei der Hund:
(a) ausgewählt ist aus der Gruppe bestehend aus einem Greyhound, einem Irischen Wolfshund, einem Basenji, einem Italienischen Windspiel, einem Galgo Español, einem Azawakh, einem Schottischen Hirschhund, einem Whippet, einem Saluki, einem Peruanischen Nackthund, einer Englischen Bulldogge, einem Shetland Sheepdog, einem Jack Russell Terrier, einer Französischen Bulldogge, einem Shiba Inu, einem Boston Terrier, einem Neufundländer, einem Akita, einem Golden Retriever, einem Alaskan Malamute, einem Australischen Treibhund, einem Boxer, einem Border Collie, einem Yorkshire Terrier, einem Cairn Terrier, einem Pyrenäen-Berghund, einem Lhasa Apso, einem Zwergschnauzer, einem Deutschen Schäferhund, einem American Staffordshire Terrier, einem Pitbull, einem Chesapeake Bay Retriever, einem Beagle, einem Collie, einem Labrador Retriever, einem Rottweiler, einem Chow Chow und einer Mischlingsrasse;
(b) ausgewählt ist aus der Gruppe bestehend aus einem Schottischen Hirschhund, einem Greyhound, einem Irischen Wolfshund und einem Windhund;
(c) ein Schottischer Hirschhund ist;
(d) ein Greyhound ist;
(e) ein Irischer Wolfshund ist; oder
(f) ein Windhund ist.

7. Veterinärmedizinische Zusammensetzung zur Verwendung nach Anspruch 4, wobei das Tier wie folgt mit der veterinärmedizinischen Zusammensetzung behandelt wird:
(a) als prophylaktische Behandlung;
(b) vor einem traumaauslösenden Ereignis bei dem Tier; oder
(c) nach einem traumaauslösenden Ereignis bei dem Tier.

8. Veterinärmedizinische Zusammensetzung zur Verwendung nach Anspruch 4, wobei die Blutungen bei dem Tier:
(a) Spätblutungen sind;
(b) Spätblutungen im Zusammenhang mit einer an dem Tier durchgeführten Operation sind; oder
(c) im Anschluss an einen operativen Eingriff erfolgen.

9. Veterinärmedizinische Zusammensetzung zur Verwendung nach Anspruch 4, wobei die genetische Mutation in dem SERPINF2-Gen darauf hinweist, dass das Tier dafür anfällig ist, Spätblutungen zu erleben.

10. Veterinärmedizinische Zusammensetzung zur Verwendung nach Anspruch 4, wobei die veterinärmedizinische Zusammensetzung einen aktiven Inhaltsstoff umfasst, der einen Plasmininhibitor umfasst.

11. Veterinärmedizinische Zusammensetzung zur Verwendung nach Anspruch 10, wobei der Plasmininhibitor:
(a) dem Tier in einer therapeutisch wirksamen Dosis verabreicht wird;
(b) dem Tier über eine Injektion verabreicht wird; oder
(c) dem Tier oral verabreicht wird.

12. Veterinärmedizinische Zusammensetzung zur Verwendung nach Anspruch 10, wobei der Plasmininhibitor:
(a) ausgewählt ist aus der Gruppe bestehend aus einer Aminocapronsäure, einer Tranexamsäure, einem Yunnan Baiyao und einer beliebigen Kombination davon;
(b) eine Aminocapronsäure ist;
(c) eine Tranexamsäure ist; oder
(d) ein Yunnan Baiyao ist.

13. Veterinärmedizinische Zusammensetzung zur Verwendung nach Anspruch 12, wobei der Plasmininhibitor:
(a) eine Aminocapronsäure ist, wobei die Aminocapronsäure in einer Dosis von 25 mg/kg bis 50 mg/kg oder in einer Dosis von 25-50 mg/kg alle 6-8 Stunden verabreicht wird;
(b) eine Tranexamsäure ist, wobei die Tranexamsäure in einer Dosis von 10 mg/kg bis 15 mg/kg oder in einer Dosis von 10 mg/kg bis 15 mg/kg alle 8-12 Stunden verabreicht wird;
(c) ein Yunnan Baiyao ist, wobei die Yunnan Baiyao in einer Dosis von 12,5 mg/kg bis 25 mg/kg oder in einer Dosis von 12,5 mg/kg bis 25 mg/kg alle 12 Stunden verabreicht wird.

## Revendications

1. Procédé de détection de la sensibilité aux hémorragies chez un animal canin, ledit procédé comprenant l'étape de :
- détermination pour savoir si une mutation génétique dans un gène SERPINF2 est présente chez l'animal, ladite présence de la mutation génétique dans le gène SERPINF2 indiquant que l'animal est sensible aux hémorragies, ladite mutation génétique dans le gène SERPINF2 étant c.605 C>T ou p.A202V.

2. Procédé selon la revendication 1, ledit procédé comprenant en outre l'étape de prise d'une décision de sélection sur la base de la présence de la mutation génétique dans le gène SERPINF2.

3. Procédé permettant de prendre une décision de sélection pour un animal canin, ledit procédé comprenant les étapes de :
- détermination pour savoir si une mutation génétique dans un gène SERPINF2 est présente chez l'animal, ladite présence de la mutation génétique dans le gène SERPINF2 indiquant que l'animal est sensible aux hémorragies, ladite mutation génétique dans le gène SERPINF2 étant c.605 C>T ou p.A202V, et
- prise de la décision de sélection sur la base de la présence de la mutation génétique dans le gène SERPINF2.

4. Composition vétérinaire destinée à être utilisée dans le traitement des hémorragies chez un animal canin dans laquelle il a été déterminé qu'une mutation génétique dans un gène SERPINF2 est présente chez l'animal, ladite mutation génétique dans le gène SERPINF2 étant c.605 C>T ou p.A202V.

5. Procédé selon la revendication 1, ladite hémorragie chez l'animal étant une hémorragie retardée.

6. Procédé selon la revendication 1 ou 3 ou composition vétérinaire destinée à être utilisée selon la revendication 4, ledit canin étant :
(a) choisi dans le groupe constitué par un lévrier greyhound, un lévrier irlandais, un basenji, un lévrier italien, un galgo espagnol, un azawakh, un lévrier écossais, un whippet, un saluki, un chien nu du Pérou, un bulldog anglais, un berger des Shetland, un Jack Russell terrier, un bulldog français, un shiba inu, un terrier de Boston, un terre-neuve, un akita, un golden retriever, un malamute d'Alaska, un bouvier australien, un boxer, un border collie, un terrier du Yorkshire, un cairn terrier, un chien de montagne des Pyrénées, un Lhasa apso, un schnauzer nain, un berger allemand, un Staffordshire terrier américain, un pitbull, un retriever de la baie de Chesapeake, un beagle, un collie, un retriever du Labrador, un rottweiler, un chow chow, et une race mixte ;
(b) choisi dans le groupe constitué par un lévrier écossais, un lévrier greyhound, un lévrier irlandais et un lévrier ;
(c) un lévrier écossais ;
(d) un lévrier greyhound ;
(e) un lévrier irlandais ; ou
(f) un lévrier.

7. Composition vétérinaire destinée à être utilisée selon la revendication 4, ledit animal étant traité avec ladite composition vétérinaire :
(a) sous la forme d'un traitement prophylactique ;
(b) avant un incident induisant un traumatisme de l'animal ; ou
(c) après un incident induisant un traumatisme de l'animal.

8. Composition vétérinaire destinée à être utilisée selon la revendication 4, ladite hémorragie chez l'animal étant :
(a) une hémorragie retardée ;
(b) un hémorragie retardé associée à une intervention chirurgicale effectuée sur l'animal ; ou
(c) à la suite d'une intervention chirurgicale.

9. Composition vétérinaire destinée à être utilisée selon la revendication 4, ladite mutation génétique dans le gène SERPINF2 indiquant que l'animal est sensible au fait de subir une hémorragie retardée.

10. Composition vétérinaire destinée à être utilisée selon la revendication 4, ladite composition vétérinaire comprenant un ingrédient actif comprenant un inhibiteur de plasmine.

11. Composition vétérinaire destinée à être utilisée selon la revendication 10, ledit inhibiteur de plasmine étant :
(a) administré à l'animal à une dose thérapeutiquement efficace ;
(b) administré à l'animal par injection ; ou
(c) administré à l'animal par voie orale.

12. Composition vétérinaire destinée à être utilisée selon la revendication 10, ledit inhibiteur de plasmine étant :
(a) choisi dans le groupe constitué par un acide aminocaproïque, un acide tranexamique, un yunnan baiyao et toute combinaison de ceux-ci ;
(b) un acide aminocaproïque ;
(c) un acide tranexamique ; ou
(d) un baiyao yunnan.

13. Composition vétérinaire destinée à être utilisée selon la revendication 12, ledit inhibiteur de plasmine étant :
(a) un acide aminocaproïque, ledit acide aminocaproïque étant administré à une dose de 25 mg/kg à 50 mg/kg ou à une dose de 25 à 50 mg/kg toutes les 6 à 8 heures ;
(b) un acide tranexamique, ledit acide tranexamique étant administré à une dose de 10 mg/kg à 15 mg/kg ou à une dose de 10 mg/kg à 15 mg/kg toutes les 8 à 12 heures ;
(c) un yunnan baiyao, ledit yunnan baiyao étant administré à une dose de 12,5 mg/kg à 25 mg/kg ou à une dose de 12,5 mg/kg à 25 mg/kg toutes les 12 heures.
